# EUROPEAN PATENT SPECIFICATION

(11) **EP 2 877 494 B1**
(45) Date of publication and mention of the grant of the patent: **15.07.2020**
(21) Application number: 13823022.2
(22) Date of filing: 23.07.2013
(51) Int. Cl.: C07K 16/40, G01N 33/573, A61K 39/395, A61P 37/00, C07K 14/705, C07K 16/18, C12N 9/16

(54) **PTPRS AND PROTEOGLYCANS IN AUTOIMMUNE DISEASE**
PTPRS UND PROTEOGLYKANE BEI AUTOIMMUNKRANKHEITEN
PTPRS ET PROTÉOGLYCANES DANS UNE MALADIE AUTO-IMMUNE

(30) Priority: 23.07.2012 US 201261674853 P; 24.07.2012 US 201261675036 P; 07.06.2013 US 201361832688 P
(43) Date of publication of application: 03.06.2015
(73) Proprietor: La Jolla Institute for Allergy and Immunology, La Jolla, CA 92037 (US)
(72) Inventor: BOTTINI, Nunzio, La Jolla, CA 92037 (US); STANFORD, Stephanie, La Jolla, CA 92037 (US)
(74) Representative: Boult Wade Tennant LLP
(86) International application number: PCT/US2013/051723
(87) International publication number: WO 2014/018554

(56) References cited:
- WO-A1-2012/148003
- WO-A2-2007/041317
- WO-A2-2011/097327
- C. H. COLES ET AL: "Proteoglycan-Specific Molecular Switch for RPTP Clustering and Neuronal Extension", SCIENCE, vol. 332, no. 6028, 31 March 2011 (2011-03-31), pages 484-488, XP055123971, ISSN: 0036-8075, DOI: 10.1126/science.1200840
- K. M. DOODY ET AL: "Targeting phosphatase-dependent proteoglycan switch for rheumatoid arthritis therapy", SCIENCE TRANSLATIONAL MEDICINE, vol. 7, no. 288, 20 May 2015 (2015-05-20), pages 288ra76-288ra76, XP055253889, Washington, DC ISSN: 1946-6234, DOI: 10.1126/scitranslmed.aaa4616
- STANFORD STEPHANIE M ET AL: "Receptor Protein Tyrosine Phosphatase [alpha]-Mediated Enhancement of Rheumatoid Synovial Fibroblast Signaling and Promotion of Arthritis in Mice.", ARTHRITIS & RHEUMATOLOGY (HOBOKEN, N.J.) FEB 2016, vol. 68, no. 2, 1 February 2016 (2016-02-01), pages 359-369, XP009188793, ISSN: 2326-5205
- STANFORD STEPHANIE M ET AL: "TGF beta responsive tyrosine phosphatase promotes rheumatoid synovial fibroblast invasiveness", ANNALS OF THE RHEUMATIC DISEASES, vol. 75, no. 1, January 2016 (2016-01), pages 295-302, XP009188802, DOI: 10.1136/ANNRHEUMDIS-2014-205790
- STEPHANIE M. STANFORD ET AL: "Protein Tyrosine Phosphatase Expression Profile of Rheumatoid Arthritis Fibroblast-like Synoviocytes: A Novel Role of SH2 Domain-Containing Phosphatase 2 as a Modulator of Invasion and Survival", ARTHRITIS & RHEUMATISM, vol. 65, no. 5, 23 May 2013 (2013-05-23), pages 1171-1180, XP055253976, US ISSN: 0004-3591, DOI: 10.1002/art.37872
- Y. SHEN ET AL: "PTP Is a Receptor for Chondroitin Sulfate Proteoglycan, an Inhibitor of Neural Regeneration", SCIENCE, vol. 326, no. 5952, 15 October 2009 (2009-10-15), pages 592-596, XP055123975, ISSN: 0036-8075, DOI: 10.1126/science.1178310
- COLES, CHARLOTTE H. ET AL.: 'Proteoglycan-specific molecular switch for RPTP clustering and neuronal extension' SCIENCE vol. 332, no. 6028, 2011, pages 484 - 488, XP055123971
- NIKOLAIENKO, ROMAN M. ET AL.: 'Receptor protein tyrosine phosphatases and cancer: new insights from structural biology' CELL ADHESION & MIGRATION vol. 6, no. 4, 01 July 2012, pages 356 - 364, XP055190228
- MOHEBIANY, ALMA N. ET AL.: 'Receptor-type tyrosine phosphatase ligands: looking for the needle in the haystack' THE FEBS JOURNAL vol. 280, no. 2, 05 July 2012, pages 388 - 400, XP055190229

### SEQUENCE LISTING

<110> La Jolla Institute of Allergy and Immunology Bottini, Nanzio Stanford, Stephanie
<120> PTPRS AND PROTEOGLYCANS IN RHEUMATOID ARTHRITIS
<130> 93138-000400PC-880314
<150> 61/674,853
   <151> 2012-07-23
<150> 61/675,036
   <151> 2012-07-24
<150> 61/832,688
   <151> 2013-06-07
<160> 13
<170> PatentIn version 3.5
<210> 1
   <211> 97
   <212> PRT
   <213> Mus musculus
<400> 1
   Glu Glu Pro Arg Phe Ile Lys Glu Pro Lys Asp Gln Ile Gly Val Ser
   1               5                   10                  15
   Gly Gly Val Ala Ser Phe Val Cys Gln Ala Thr Gly Asp Pro Lys Pro
               20                  25                  30
   Arg Val Thr Trp Asn Lys Lys Gly Lys Lys Val Asn Ser Gln Arg Phe
           35                  40                  45
   Glu Thr Ile Glu Phe Asp Glu Ser Ala Gly Ala Val Leu Arg Ile Gln
       50                  55                  60
   Pro Leu Arg Thr Pro Arg Asp Glu Asn Val Tyr Glu Cys Val Ala Gln
   65                  70                  75                  80
   Asn Ser Val Gly Glu Ile Thr Val His Ala Lys Leu Thr Val Leu Arg
                   85                  90                  95
   Glu
<210> 2
   <211> 113
   <212> PRT
   <213> Mus musculus
<400> 2
   Asp Gln Leu Pro Ser Gly Phe Pro Asn Ile Asp Met Gly Pro Gln Leu
   1               5                   10                  15
   Lys Val Val Glu Arg Thr Arg Thr Ala Thr Met Leu Cys Ala Ala Ser
               20                  25                  30
   Gly Asn Pro Asp Pro Glu Ile Thr Trp Phe Lys Asp Phe Leu Pro Val
           35                  40                  45
   Asp Pro Ser Ala Ser Asn Gly Arg Ile Lys Gln Leu Arg Ser Glu Thr
       50                  55                  60
   Phe Glu Ser Thr Pro Ile Arg Gly Ala Leu Gln Ile Glu Ser Ser Glu
   65                  70                  75                  80
   Glu Thr Asp Gln Gly Lys Tyr Glu Cys Val Ala Thr Asn Ser Ala Gly
                   85                  90                  95
   Val Arg Tyr Ser Ser Pro Ala Asn Leu Tyr Val Arg Val Arg Arg Val
               100                 105                 110
   Ala
<210> 3
   <211> 90
   <212> PRT
   <213> Mus musculus
<400> 3
   Pro Arg Phe Ser Ile Leu Pro Met Ser His Glu Ile Met Pro Gly Gly
   1               5                   10                  15
   Asn Val Asn Ile Thr Cys Val Ala Val Gly Ser Pro Met Pro Tyr Val
               20                  25                  30
   Lys Trp Met Gln Gly Ala Glu Asp Leu Thr Pro Glu Asp Asp Met Pro
           35                  40                  45
   Val Gly Arg Asn Val Leu Glu Leu Thr Asp Val Lys Asp Ser Ala Asn
       50                  55                  60
   Tyr Thr Cys Val Ala Met Ser Ser Leu Gly Val Ile Glu Ala Val Ala
   65                  70                  75                  80
   Gln Ile Thr Val Lys Ser Leu Pro Lys Ala
                   85                  90
<210> 4
   <211> 1907
   <212> PRT
   <213> Mus musculus
<400> 4
   Met Ala Pro Thr Trp Ser Pro Ser Val Val Ser Val Val Gly Pro Val
   1               5                   10                  15
   Gly Leu Phe Leu Val Leu Leu Ala Arg Gly Cys Leu Ala Glu Glu Pro
               20                  25                  30
   Pro Arg Phe Ile Arg Glu Pro Lys Asp Gln Ile Gly Val Ser Gly Gly
           35                  40                  45
   Val Ala Ser Phe Val Cys Gln Ala Thr Gly Asp Pro Lys Pro Arg Val
       50                  55                  60
   Thr Trp Asn Lys Lys Gly Lys Lys Val Asn Ser Gln Arg Phe Glu Thr
   65                  70                  75                  80
   Ile Asp Phe Asp Glu Ser Ser Gly Ala Val Leu Arg Ile Gln Pro Leu
                   85                  90                  95
   Arg Thr Pro Arg Asp Glu Asn Val Tyr Glu Cys Val Ala Gln Asn Ser
               100                 105                 110
   Val Gly Glu Ile Thr Ile His Ala Lys Leu Thr Val Leu Arg Glu Asp
           115                 120                 125
   Gln Leu Pro Pro Gly Phe Pro Asn Ile Asp Met Gly Pro Gln Leu Lys
       130                 135                 140
   Val Val Glu Arg Thr Arg Thr Ala Thr Met Leu Cys Ala Ala Ser Gly
   145                 150                 155                 160
   Asn Pro Asp Pro Glu Ile Thr Trp Phe Lys Asp Phe Leu Pro Val Asp
                   165                 170                 175
   Pro Ser Ala Ser Asn Gly Arg Ile Lys Gln Leu Arg Ser Gly Ala Leu
               180                 185                 190
   Gln Ile Glu Ser Ser Glu Glu Thr Asp Gln Gly Lys Tyr Glu Cys Val
           195                 200                 205
   Ala Thr Asn Ser Ala Gly Val Arg Tyr Ser Ser Pro Ala Asn Leu Tyr
       210                 215                 220
   Val Arg Val Arg Arg Val Ala Pro Arg Phe Ser Ile Leu Pro Met Ser
   225                 230                 235                 240
   His Glu Ile Met Pro Gly Gly Asn Val Asn Ile Thr Cys Val Ala Val
                   245                 250                 255
   Gly Ser Pro Met Pro Tyr Val Lys Trp Met Gln Gly Ala Glu Asp Leu
               260                 265                 270
   Thr Pro Glu Asp Asp Met Pro Val Gly Arg Asn Val Leu Glu Leu Thr
           275                 280                 285
   Asp Val Lys Asp Ser Ala Asn Tyr Thr Cys Val Ala Met Ser Ser Leu
       290                 295                 300
   Gly Val Ile Glu Ala Val Ala Gln Ile Thr Val Lys Ser Leu Pro Lys
   305                 310                 315                 320
   Ala Pro Gly Thr Pro Val Val Thr Glu Asn Thr Ala Thr Ser Ile Thr
                   325                 330                 335
   Val Thr Trp Asp Ser Gly Asn Pro Asp Pro Val Ser Tyr Tyr Val Ile
               340                 345                 350
   Glu Tyr Lys Ser Lys Ser Gln Asp Gly Pro Tyr Gln Ile Lys Glu Asp
           355                 360                 365
   Ile Thr Thr Thr Arg Tyr Ser Ile Gly Gly Leu Ser Pro Asn Ser Glu
       370                 375                 380
   Tyr Glu Ile Trp Val Ser Ala Val Asn Ser Ile Gly Gln Gly Pro Pro
   385                 390                 395                 400
   Ser Glu Ser Val Val Thr Arg Thr Gly Glu Gln Ala Pro Ala Ser Ala
                   405                 410                 415
   Pro Arg Asn Val Gln Ala Arg Met Leu Ser Ala Thr Thr Met Ile Val
               420                 425                 430
   Gln Trp Glu Glu Pro Val Glu Pro Asn Gly Leu Ile Arg Gly Tyr Arg
           435                 440                 445
   Val Tyr Tyr Thr Met Glu Pro Glu His Pro Val Gly Asn Trp Gln Lys
       450                 455                 460
   His Asn Val Asp Asp Ser Leu Leu Thr Thr Val Gly Ser Leu Leu Glu
   465 470 475 480
   Asp Glu Thr Tyr Thr Val Arg Val Leu Ala Phe Thr Ser Val Gly Asp
   485 490 495
   Gly Pro Leu Ser Asp Pro Ile Gln Val Lys Thr Gln Gln Gly Val Pro
   500 505 510
   Gly Gln Pro Met Asn Leu Arg Ala Glu Ala Lys Ser Glu Thr Ser Ile
   515 520 525
   Gly Leu Ser Trp Ser Ala Pro Arg Gln Glu Ser Val Ile Lys Tyr Glu
   530 535 540
   Leu Leu Phe Arg Glu Gly Asp Arg Gly Arg Glu Val Gly Arg Thr Phe
   545 550 555 560
   Asp Pro Thr Thr Ala Phe Val Val Glu Asp Leu Lys Pro Asn Thr Glu
   565 570 575
   Tyr Ala Phe Arg Leu Ala Ala Arg Ser Pro Gln Gly Leu Gly Ala Phe
   580 585 590
   Thr Ala Val Val Arg Gln Arg Thr Leu Gln Ala Lys Pro Ser Ala Pro
   595 600 605
   Pro Gln Asp Val Lys Cys Thr Ser Leu Arg Ser Thr Ala Ile Leu Val
   610 615 620
   Ser Trp Arg Pro Pro Pro Pro Glu Thr His Asn Gly Ala Leu Val Gly
   625 630 635 640
   Tyr Ser Val Arg Tyr Arg Pro Leu Gly Ser Glu Asp Pro Asp Pro Lys
   645 650 655
   Glu Val Asn Asn Ile Pro Pro Thr Thr Thr Gln Ile Leu Leu Glu Ala
   660 665 670
   Leu Glu Lys Trp Thr Glu Tyr Arg Val Thr Ala Val Ala Tyr Thr Glu
   675 680 685
   Val Gly Pro Gly Pro Glu Ser Ser Pro Val Val Val Arg Thr Asp Glu
   690 695 700
   Asp Val Pro Ser Ala Pro Pro Arg Lys Val Glu Ala Glu Ala Leu Asn
   705 710 715 720
   Ala Thr Ala Ile Arg Val Leu Trp Arg Ser Pro Thr Pro Gly Arg Gln
                   725                 730                 735
   His Gly Gln Ile Arg Gly Tyr Gln Val His Tyr Val Arg Met Glu Gly
               740                 745                 750
   Ala Glu Ala Arg Gly Pro Pro Arg Ile Lys Asp Ile Met Leu Ala Asp
           755                 760                 765
   Ala Gln Glu Met Val Ile Thr Asn Leu Gln Pro Glu Thr Ala Tyr Ser
       770                 775                 780
   Ile Thr Val Ala Ala Tyr Thr Met Lys Gly Asp Gly Ala Arg Ser Lys
   785                 790                 795                 800
   Pro Lys Val Val Val Thr Lys Gly Ala Val Leu Gly Arg Pro Thr Leu
                   805                 810                 815
   Ser Val Gln Gln Thr Pro Glu Gly Ser Leu Leu Ala Arg Trp Glu Pro
               820                 825                 830
   Pro Ala Asp Ala Ala Glu Asp Pro Val Leu Gly Tyr Arg Leu Gln Phe
           835                 840                 845
   Gly Arg Glu Asp Ala Ala Pro Ala Thr Leu Glu Leu Ala Ala Trp Glu
       850                 855                 860
   Arg Arg Phe Ala Ala Pro Ala His Lys Gly Ala Thr Tyr Val Phe Arg
   865                 870                 875                 880
   Leu Ala Ala Arg Gly Arg Ala Gly Leu Gly Glu Glu Ala Ala Ala Ala
                   885                 890                 895
   Leu Ser Ile Pro Glu Asp Ala Pro Arg Gly Phe Pro Gln Ile Leu Gly
               900                 905                 910
   Ala Ala Gly Asn Val Ser Ala Gly Ser Val Leu Leu Arg Trp Leu Pro
           915                 920                 925
   Pro Val Pro Ala Glu Arg Asn Gly Ala Ile Ile Lys Tyr Thr Val Ser
       930                 935                 940
   Val Arg Glu Ala Gly Ala Pro Gly Pro Ala Thr Glu Thr Glu Leu Ala
   945                 950                 955                 960
   Ala Ala Ala Gln Pro Gly Ala Glu Thr Ala Leu Thr Leu Arg Gly Leu
                   965                 970                 975
   Arg Pro Glu Thr Ala Tyr Glu Leu Arg Val Arg Ala His Thr Arg Arg
               980                 985                 990
   Gly Pro Gly Pro Phe Ser Pro Pro  Leu Arg Tyr Arg Leu  Ala Arg Asp
           995                 1000                 1005
   Pro Val  Ser Pro Lys Asn Phe  Lys Val Lys Met Ile  Met Lys Thr
       1010                 1015                 1020
   Ser Val  Leu Leu Ser Trp Glu  Phe Pro Asp Asn Tyr  Asn Ser Pro
       1025                 1030                 1035
   Thr Pro  Tyr Lys Ile Gln Tyr  Asn Gly Leu Thr Leu  Asp Val Asp
       1040                 1045                 1050
   Gly Arg  Thr Thr Lys Lys Leu  Ile Thr His Leu Lys  Pro His Thr
       1055                 1060                 1065
   Phe Tyr  Asn Phe Val Leu Thr  Asn Arg Gly Ser Ser  Leu Gly Gly
       1070                 1075                 1080
   Leu Gln  Gln Thr Val Thr Ala  Arg Thr Ala Phe Asn  Met Leu Ser
       1085                 1090                 1095
   Gly Lys  Pro Ser Val Ala Pro  Lys Pro Asp Asn Asp  Gly Phe Ile
       1100                 1105                 1110
   Val Val  Tyr Leu Pro Asp Gly  Gln Ser Pro Val Thr  Val Gln Asn
       1115                 1120                 1125
   Tyr Phe  Ile Val Met Val Pro  Leu Arg Lys Ser Arg  Gly Gly Gln
       1130                 1135                 1140
   Phe Pro  Val Leu Leu Gly Ser  Pro Glu Asp Met Asp  Leu Glu Glu
       1145                 1150                 1155
   Leu Ile  Gln Asp Ile Ser Arg  Leu Gln Arg Arg Ser  Leu Arg His
       1160                 1165                 1170
   Ser Arg  Gln Leu Glu Val Pro  Arg Pro Tyr Ile Ala  Ala Arg Phe
       1175                 1180                 1185
   Ser Ile  Leu Pro Ala Val Phe  His Pro Gly Asn Gln  Lys Gln Tyr
       1190                 1195                 1200
   Gly Gly  Phe Asp Asn Arg Gly  Leu Glu Pro Gly His  Arg Tyr Val
       1205                 1210                 1215
   Leu Phe  Val Leu Ala Val Leu  Gln Lys Asn Glu Pro  Thr Phe Ala
       1220                 1225                 1230
   Ala Ser  Pro Phe Ser Asp Pro  Phe Gln Leu Asp Asn  Pro Asp Pro
       1235                 1240                 1245
   Gln Pro  Ile Val Asp Gly Glu  Glu Gly Leu Ile Trp  Val Ile Gly
       1250                 1255                 1260
   Pro Val  Leu Ala Val Val Phe  Ile Ile Cys Ile Val  Ile Ala Ile
       1265                 1270                 1275
   Leu Leu  Tyr Lys Asn Lys Pro  Asp Ser Lys Arg Lys  Asp Ser Glu
       1280                 1285                 1290
   Pro Arg  Thr Lys Cys Leu Leu  Asn Asn Ala Asp Leu  Ala Pro His
       1295                 1300                 1305
   His Pro  Lys Asp Pro Val Glu  Met Arg Arg Ile Asn  Phe Gln Thr
       1310                 1315                 1320
   Pro Gly  Met Leu Ser His Pro  Pro Ile Pro Ile Thr  Asp Met Ala
       1325                 1330                 1335
   Glu His  Met Glu Arg Leu Lys  Ala Asn Asp Ser Leu  Lys Leu Ser
       1340                 1345                 1350
   Gln Glu  Tyr Glu Ser Ile Asp  Pro Gly Gln Gln Phe  Thr Trp Glu
       1355                 1360                 1365
   His Ser  Asn Leu Glu Ala Asn  Lys Pro Lys Asn Arg  Tyr Ala Asn
       1370                 1375                 1380
   Val Ile  Ala Tyr Asp His Ser  Arg Val Ile Leu Gln  Pro Leu Glu
       1385                 1390                 1395
   Gly Ile  Met Gly Ser Asp Tyr  Ile Asn Ala Asn Tyr  Val Asp Gly
       1400                 1405                 1410
   Tyr Arg  Arg Gln Asn Ala Tyr  Ile Ala Thr Gln Gly  Pro Leu Pro
       1415                 1420                 1425
   Glu Thr  Phe Gly Asp Phe Trp  Arg Met Val Trp Glu  Gln Arg Ser
       1430                 1435                 1440
   Ala Thr  Val Val Met Met Thr  Arg Leu Glu Glu Lys  Ser Arg Ile
   1445 1450 1455
   Lys Cys Asp Gln Tyr Trp Pro Asn Arg Gly Thr Glu Thr Tyr Gly
   1460 1465 1470
   Phe Ile Gln Val Thr Leu Leu Asp Thr Met Glu Leu Ala Thr Phe
   1475 1480 1485
   Cys Val Arg Thr Phe Ser Leu His Lys Asn Gly Ser Ser Glu Lys
   1490 1495 1500
   Arg Glu Val Arg His Phe Gln Phe Thr Ala Trp Pro Asp His Gly
   1505 1510 1515
   Val Pro Glu Tyr Pro Thr Pro Phe Leu Ala Phe Leu Arg Arg Val
   1520 1525 1530
   Lys Thr Cys Asn Pro Pro Asp Ala Gly Pro Ile Val Val His Cys
   1535 1540 1545
   Ser Ala Gly Val Gly Arg Thr Gly Cys Phe Ile Val Ile Asp Ala
   1550 1555 1560
   Met Leu Glu Arg Ile Lys Thr Glu Lys Thr Val Asp Val Tyr Gly
   1565 1570 1575
   His Val Thr Leu Met Arg Ser Gln Arg Asn Tyr Met Val Gln Thr
   1580 1585 1590
   Glu Asp Gln Tyr Gly Phe Ile His Glu Ala Leu Leu Glu Ala Val
   1595 1600 1605
   Gly Cys Gly Asn Thr Glu Val Pro Ala Arg Ser Leu Tyr Thr Tyr
   1610 1615 1620
   Ile Gln Lys Leu Ala Gln Val Glu Pro Gly Glu His Val Thr Gly
   1625 1630 1635
   Met Glu Leu Glu Phe Lys Arg Leu Ala Ser Ser Lys Ala His Thr
   1640 1645 1650
   Ser Arg Phe Ile Thr Ala Ser Leu Pro Cys Asn Lys Phe Lys Asn
   1655 1660 1665
   Arg Leu Val Asn Ile Leu Pro Tyr Glu Ser Ser Arg Val Cys Leu
   1670 1675 1680
   Gln Pro Ile Arg Gly Val Glu Gly Ser Asp Tyr Ile Asn Ala Ser
       1685                1690                1695
   Phe Ile Asp Gly Tyr Arg Gln Gln Lys Ala Tyr Ile Ala Thr Gln
       1700                1705                1710
   Gly Pro Leu Ala Glu Thr Thr Glu Asp Phe Trp Arg Ala Leu Trp
       1715                1720                1725
   Glu Asn Asn Ser Thr Ile Val Val Met Leu Thr Lys Leu Arg Glu
       1730                1735                1740
   Met Gly Arg Glu Lys Cys His Gln Tyr Trp Pro Ala Glu Arg Ser
       1745                1750                1755
   Ala Arg Tyr Gln Tyr Phe Val Val Asp Pro Met Ala Glu Tyr Asn
       1760                1765                1770
   Met Pro Gln Tyr Ile Leu Arg Glu Phe Lys Val Thr Asp Ala Arg
       1775                1780                1785
   Asp Gly Gln Ser Arg Thr Val Arg Gln Phe Gln Phe Thr Asp Trp
       1790                1795                1800
   Pro Glu Gln Gly Ala Pro Lys Ser Gly Glu Gly Phe Ile Asp Phe
       1805                1810                1815
   Ile Gly Gln Val His Lys Thr Lys Glu Gln Phe Gly Gln Asp Gly
       1820                1825                1830
   Pro Ile Ser Val His Cys Ser Ala Gly Val Gly Arg Thr Gly Val
       1835                1840                1845
   Phe Ile Thr Leu Ser Ile Val Leu Glu Arg Met Arg Tyr Glu Gly
       1850                1855                1860
   Val Val Asp Ile Phe Gln Thr Val Lys Val Leu Arg Thr Gln Arg
       1865                1870                1875
   Pro Ala Met Val Gln Thr Glu Asp Glu Tyr Gln Phe Cys Phe Gln
       1880                1885                1890
   Ala Ala Leu Glu Tyr Leu Gly Ser Phe Asp His Tyr Ala Thr
       1895                1900                1905
<210> 5
   <211> 98
   <212> PRT
   <213> Homo sapiens
<400> 5
   Glu Glu Pro Pro Arg Phe Ile Lys Glu Pro Lys Asp Gln Ile Gly Val
   1               5                   10                  15
   Ser Gly Gly Val Ala Ser Phe Val Cys Gln Ala Thr Gly Asp Pro Lys
               20                  25                  30
   Pro Arg Val Thr Trp Asn Lys Lys Gly Lys Lys Val Asn Ser Gln Arg
           35                  40                  45
   Phe Glu Thr Ile Glu Phe Asp Glu Ser Ala Gly Ala Val Leu Arg Ile
       50                  55                  60
   Gln Pro Leu Arg Thr Pro Arg Asp Glu Asn Val Tyr Glu Cys Val Ala
   65                  70                  75                  80
   Gln Asn Ser Val Gly Glu Ile Thr Val His Ala Lys Leu Thr Val Leu
                   85                  90                  95
   Arg Glu
<210> 6
   <211> 117
   <212> PRT
   <213> Homo sapiens
<400> 6
   Asp Gln Leu Pro Ser Gly Phe Pro Asn Ile Asp Met Gly Pro Gln Leu
   1               5                   10                  15
   Lys Val Val Glu Arg Thr Arg Thr Ala Thr Met Leu Cys Ala Ala Ser
               20                  25                  30
   Gly Asn Pro Asp Pro Glu Ile Thr Trp Phe Lys Asp Phe Leu Pro Val
           35                  40                  45
   Asp Pro Ser Ala Ser Asn Gly Arg Ile Lys Gln Leu Arg Ser Glu Thr
       50                  55                  60
   Phe Glu Ser Thr Pro Ile Arg Gly Ala Leu Gln Ile Glu Ser Ser Glu
   65                  70                  75                  80
   Glu Thr Asp Gln Gly Lys Tyr Glu Cys Val Ala Thr Asn Ser Ala Gly
                   85                  90                  95
   Val Arg Tyr Ser Ser Pro Ala Asn Leu Tyr Val Arg Glu Leu Arg Glu
               100                 105                 110
   Val Arg Arg Val Ala
           115
<210> 7
   <211> 90
   <212> PRT
   <213> Homo sapiens
<400> 7
   Pro Arg Phe Ser Ile Leu Pro Met Ser His Glu Ile Met Pro Gly Gly
   1               5                   10                  15
   Asn Val Asn Ile Thr Cys Val Ala Val Gly Ser Pro Met Pro Tyr Val
               20                  25                  30
   Lys Trp Met Gln Gly Ala Glu Asp Leu Thr Pro Glu Asp Asp Met Pro
           35                  40                  45
   Val Gly Arg Asn Val Leu Glu Leu Thr Asp Val Lys Asp Ser Ala Asn
       50                  55                  60
   Tyr Thr Cys Val Ala Met Ser Ser Leu Gly Val Ile Glu Ala Val Ala
   65                  70                  75                  80
   Gln Ile Thr Val Lys Ser Leu Pro Lys Ala
                   85                  90
<210> 8
   <211> 1948
   <212> PRT
   <213> Homo sapiens
<400> 8
   Met Ala Pro Thr Trp Gly Pro Gly Met Val Ser Val Val Gly Pro Met
   1               5                   10                  15
   Gly Leu Leu Val Val Leu Leu Val Gly Gly Cys Ala Ala Glu Glu Pro
               20                  25                  30
   Pro Arg Phe Ile Lys Glu Pro Lys Asp Gln Ile Gly Val Ser Gly Gly
           35                  40                  45
   Val Ala Ser Phe Val Cys Gln Ala Thr Gly Asp Pro Lys Pro Arg Val
       50                  55                  60
   Thr Trp Asn Lys Lys Gly Lys Lys Val Asn Ser Gln Arg Phe Glu Thr
   65 70 75 80
   Ile Glu Phe Asp Glu Ser Ala Gly Ala Val Leu Arg Ile Gln Pro Leu
   85 90 95
   Arg Thr Pro Arg Asp Glu Asn Val Tyr Glu Cys Val Ala Gln Asn Ser
   100 105 110
   Val Gly Glu Ile Thr Val His Ala Lys Leu Thr Val Leu Arg Glu Asp
   115 120 125
   Gln Leu Pro Ser Gly Phe Pro Asn Ile Asp Met Gly Pro Gln Leu Lys
   130 135 140
   Val Val Glu Arg Thr Arg Thr Ala Thr Met Leu Cys Ala Ala Ser Gly
   145 150 155 160
   Asn Pro Asp Pro Glu Ile Thr Trp Phe Lys Asp Phe Leu Pro Val Asp
   165 170 175
   Pro Ser Ala Ser Asn Gly Arg Ile Lys Gln Leu Arg Ser Glu Thr Phe
   180 185 190
   Glu Ser Thr Pro Ile Arg Gly Ala Leu Gln Ile Glu Ser Ser Glu Glu
   195 200 205
   Thr Asp Gln Gly Lys Tyr Glu Cys Val Ala Thr Asn Ser Ala Gly Val
   210 215 220
   Arg Tyr Ser Ser Pro Ala Asn Leu Tyr Val Arg Glu Leu Arg Glu Val
   225 230 235 240
   Arg Arg Val Ala Pro Arg Phe Ser Ile Leu Pro Met Ser His Glu Ile
   245 250 255
   Met Pro Gly Gly Asn Val Asn Ile Thr Cys Val Ala Val Gly Ser Pro
   260 265 270
   Met Pro Tyr Val Lys Trp Met Gln Gly Ala Glu Asp Leu Thr Pro Glu
   275 280 285
   Asp Asp Met Pro Val Gly Arg Asn Val Leu Glu Leu Thr Asp Val Lys
   290 295 300
   Asp Ser Ala Asn Tyr Thr Cys Val Ala Met Ser Ser Leu Gly Val Ile
   305 310 315 320
   Glu Ala Val Ala Gln Ile Thr Val Lys Ser Leu Pro Lys Ala Pro Gly
                   325                 330                 335
   Thr Pro Met Val Thr Glu Asn Thr Ala Thr Ser Ile Thr Ile Thr Trp
               340                 345                 350
   Asp Ser Gly Asn Pro Asp Pro Val Ser Tyr Tyr Val Ile Glu Tyr Lys
           355                 360                 365
   Ser Lys Ser Gln Asp Gly Pro Tyr Gln Ile Lys Glu Asp Ile Thr Thr
       370                 375                 380
   Thr Arg Tyr Ser Ile Gly Gly Leu Ser Pro Asn Ser Glu Tyr Glu Ile
   385                 390                 395                 400
   Trp Val Ser Ala Val Asn Ser Ile Gly Gln Gly Pro Pro Ser Glu Ser
                   405                 410                 415
   Val Val Thr Arg Thr Gly Glu Gln Ala Pro Ala Ser Ala Pro Arg Asn
               420                 425                 430
   Val Gln Ala Arg Met Leu Ser Ala Thr Thr Met Ile Val Gln Trp Glu
           435                 440                 445
   Glu Pro Val Glu Pro Asn Gly Leu Ile Arg Gly Tyr Arg Val Tyr Tyr
       450                 455                 460
   Thr Met Glu Pro Glu His Pro Val Gly Asn Trp Gln Lys His Asn Val
   465                 470                 475                 480
   Asp Asp Ser Leu Leu Thr Thr Val Gly Ser Leu Leu Glu Asp Glu Thr
                   485                 490                 495
   Tyr Thr Val Arg Val Leu Ala Phe Thr Ser Val Gly Asp Gly Pro Leu
               500                 505                 510
   Ser Asp Pro Ile Gln Val Lys Thr Gln Gln Gly Val Pro Gly Gln Pro
           515                 520                 525
   Met Asn Leu Arg Ala Glu Ala Arg Ser Glu Thr Ser Ile Thr Leu Ser
       530                 535                 540
   Trp Ser Pro Pro Arg Gln Glu Ser Ile Ile Lys Tyr Glu Leu Leu Phe
   545                 550                 555                 560
   Arg Glu Gly Asp His Gly Arg Glu Val Gly Arg Thr Phe Asp Pro Thr
                   565                 570                 575
   Thr Ser Tyr Val Val Glu Asp Leu Lys Pro Asn Thr Glu Tyr Ala Phe
               580                 585                 590
   Arg Leu Ala Ala Arg Ser Pro Gln Gly Leu Gly Ala Phe Thr Pro Val
           595                 600                 605
   Val Arg Gln Arg Thr Leu Gln Ser Lys Pro Ser Ala Pro Pro Gln Asp
       610                 615                 620
   Val Lys Cys Val Ser Val Arg Ser Thr Ala Ile Leu Val Ser Trp Arg
   625                 630                 635                 640
   Pro Pro Pro Pro Glu Thr His Asn Gly Ala Leu Val Gly Tyr Ser Val
                   645                 650                 655
   Arg Tyr Arg Pro Leu Gly Ser Glu Asp Pro Glu Pro Lys Glu Val Asn
               660                 665                 670
   Gly Ile Pro Pro Thr Thr Thr Gln Ile Leu Leu Glu Ala Leu Glu Lys
           675                 680                 685
   Trp Thr Gln Tyr Arg Ile Thr Thr Val Ala His Thr Glu Val Gly Pro
       690                 695                 700
   Gly Pro Glu Ser Ser Pro Val Val Val Arg Thr Asp Glu Asp Val Pro
   705                 710                 715                 720
   Ser Ala Pro Pro Arg Lys Val Glu Ala Glu Ala Leu Asn Ala Thr Ala
                   725                 730                 735
   Ile Arg Val Leu Trp Arg Ser Pro Ala Pro Gly Arg Gln His Gly Gln
               740                 745                 750
   Ile Arg Gly Tyr Gln Val His Tyr Val Arg Met Glu Gly Ala Glu Ala
           755                 760                 765
   Arg Gly Pro Pro Arg Ile Lys Asp Val Met Leu Ala Asp Ala Gln Trp
       770                 775                 780
   Glu Thr Asp Asp Thr Ala Glu Tyr Glu Met Val Ile Thr Asn Leu Gln
   785                 790                 795                 800
   Pro Glu Thr Ala Tyr Ser Ile Thr Val Ala Ala Tyr Thr Met Lys Gly
                   805                 810                 815
   Asp Gly Ala Arg Ser Lys Pro Lys Val Val Val Thr Lys Gly Ala Val
               820                 825                 830
   Leu Gly Arg Pro Thr Leu Ser Val Gln Gln Thr Pro Glu Gly Ser Leu
   835 840 845
   Leu Ala Arg Trp Glu Pro Pro Ala Gly Thr Ala Glu Asp Gln Val Leu
   850 855 860
   Gly Tyr Arg Leu Gln Phe Gly Arg Glu Asp Ser Thr Pro Leu Ala Thr
   865 870 875 880
   Leu Glu Phe Pro Pro Ser Glu Asp Arg Tyr Thr Ala Ser Gly Val His
   885 890 895
   Lys Gly Ala Thr Tyr Val Phe Arg Leu Ala Ala Arg Ser Arg Gly Gly
   900 905 910
   Leu Gly Glu Glu Ala Ala Glu Val Leu Ser Ile Pro Glu Asp Thr Pro
   915 920 925
   Arg Gly His Pro Gln Ile Leu Glu Ala Ala Gly Asn Ala Ser Ala Gly
   930 935 940
   Thr Val Leu Leu Arg Trp Leu Pro Pro Val Pro Ala Glu Arg Asn Gly
   945 950 955 960
   Ala Ile Val Lys Tyr Thr Val Ala Val Arg Glu Ala Gly Ala Leu Gly
   965 970 975
   Pro Ala Arg Glu Thr Glu Leu Pro Ala Ala Ala Glu Pro Gly Ala Glu
   980 985 990
   Asn Ala Leu Thr Leu Gln Gly Leu Lys Pro Asp Thr Ala Tyr Asp Leu
   995 1000 1005
   Gln Val Arg Ala His Thr Arg Arg Gly Pro Gly Pro Phe Ser Pro
   1010 1015 1020
   Pro Val Arg Tyr Arg Thr Phe Leu Arg Asp Gln Val Ser Pro Lys
   1025 1030 1035
   Asn Phe Lys Val Lys Met Ile Met Lys Thr Ser Val Leu Leu Ser
   1040 1045 1050
   Trp Glu Phe Pro Asp Asn Tyr Asn Ser Pro Thr Pro Tyr Lys Ile
   1055 1060 1065
   Gln Tyr Asn Gly Leu Thr Leu Asp Val Asp Gly Arg Thr Thr Lys
   1070 1075 1080
   Lys Leu Ile Thr His Leu Lys Pro His Thr Phe Tyr Asn Phe Val
   1085 1090 1095
   Leu Thr Asn Arg Gly Ser Ser Leu Gly Gly Leu Gln Gln Thr Val
   1100 1105 1110
   Thr Ala Trp Thr Ala Phe Asn Leu Leu Asn Gly Lys Pro Ser Val
   1115 1120 1125
   Ala Pro Lys Pro Asp Ala Asp Gly Phe Ile Met Val Tyr Leu Pro
   1130 1135 1140
   Asp Gly Gln Ser Pro Val Pro Val Gln Ser Tyr Phe Ile Val Met
   1145 1150 1155
   Val Pro Leu Arg Lys Ser Arg Gly Gly Gln Phe Leu Thr Pro Leu
   1160 1165 1170
   Gly Ser Pro Glu Asp Met Asp Leu Glu Glu Leu Ile Gln Asp Ile
   1175 1180 1185
   Ser Arg Leu Gln Arg Arg Ser Leu Arg His Ser Arg Gln Leu Glu
   1190 1195 1200
   Val Pro Arg Pro Tyr Ile Ala Ala Arg Phe Ser Val Leu Pro Pro
   1205 1210 1215
   Thr Phe His Pro Gly Asp Gln Lys Gln Tyr Gly Gly Phe Asp Asn
   1220 1225 1230
   Arg Gly Leu Glu Pro Gly His Arg Tyr Val Leu Phe Val Leu Ala
   1235 1240 1245
   Val Leu Gln Lys Ser Glu Pro Thr Phe Ala Ala Ser Pro Phe Ser
   1250 1255 1260
   Asp Pro Phe Gln Leu Asp Asn Pro Asp Pro Gln Pro Ile Val Asp
   1265 1270 1275
   Gly Glu Glu Gly Leu Ile Trp Val Ile Gly Pro Val Leu Ala Val
   1280 1285 1290
   Val Phe Ile Ile Cys Ile Val Ile Ala Ile Leu Leu Tyr Lys Asn
   1295 1300 1305
   Lys Pro  Asp Ser Lys Arg Lys  Asp Ser Glu Pro Arg  Thr Lys Cys
       1310                 1315                 1320
   Leu Leu  Asn Asn Ala Asp Leu  Ala Pro His His Pro  Lys Asp Pro
       1325                 1330                 1335
   Val Glu  Met Arg Arg Ile Asn  Phe Gln Thr Pro Asp  Ser Gly Leu
       1340                 1345                 1350
   Arg Ser  Pro Leu Arg Glu Pro  Gly Phe His Phe Glu  Ser Met Leu
       1355                 1360                 1365
   Ser His  Pro Pro Ile Pro Ile  Ala Asp Met Ala Glu  His Thr Glu
       1370                 1375                 1380
   Arg Leu  Lys Ala Asn Asp Ser  Leu Lys Leu Ser Gln  Glu Tyr Glu
       1385                 1390                 1395
   Ser Ile  Asp Pro Gly Gln Gln  Phe Thr Trp Glu His  Ser Asn Leu
       1400                 1405                 1410
   Glu Val  Asn Lys Pro Lys Asn  Arg Tyr Ala Asn Val  Ile Ala Tyr
       1415                 1420                 1425
   Asp His  Ser Arg Val Ile Leu  Gln Pro Ile Glu Gly  Ile Met Gly
       1430                 1435                 1440
   Ser Asp  Tyr Ile Asn Ala Asn  Tyr Val Asp Gly Tyr  Arg Cys Gln
       1445                 1450                 1455
   Asn Ala  Tyr Ile Ala Thr Gln  Gly Pro Leu Pro Glu  Thr Phe Gly
       1460                 1465                 1470
   Asp Phe  Trp Arg Met Val Trp  Glu Gln Arg Ser Ala  Thr Ile Val
       1475                 1480                 1485
   Met Met  Thr Arg Leu Glu Glu  Lys Ser Arg Ile Lys  Cys Asp Gln
       1490                 1495                 1500
   Tyr Trp  Pro Asn Arg Gly Thr  Glu Thr Tyr Gly Phe  Ile Gln Val
       1505                 1510                 1515
   Thr Leu  Leu Asp Thr Ile Glu  Leu Ala Thr Phe Cys  Val Arg Thr
       1520                 1525                 1530
   Phe Ser  Leu His Lys Asn Gly  Ser Ser Glu Lys Arg  Glu Val Arg
       1535                 1540                 1545
   Gln Phe Gln Phe Thr Ala Trp Pro Asp His Gly Val Pro Glu Tyr
   1550 1555 1560
   Pro Thr Pro Phe Leu Ala Phe Leu Arg Arg Val Lys Thr Cys Asn
   1565 1570 1575
   Pro Pro Asp Ala Gly Pro Ile Val Val His Cys Ser Ala Gly Val
   1580 1585 1590
   Gly Arg Thr Gly Cys Phe Ile Val Ile Asp Ala Met Leu Glu Arg
   1595 1600 1605
   Ile Lys Pro Glu Lys Thr Val Asp Val Tyr Gly His Val Thr Leu
   1610 1615 1620
   Met Arg Ser Gln Arg Asn Tyr Met Val Gln Thr Glu Asp Gln Tyr
   1625 1630 1635
   Ser Phe Ile His Glu Ala Leu Leu Glu Ala Val Gly Cys Gly Asn
   1640 1645 1650
   Thr Glu Val Pro Ala Arg Ser Leu Tyr Ala Tyr Ile Gln Lys Leu
   1655 1660 1665
   Ala Gln Val Glu Pro Gly Glu His Val Thr Gly Met Glu Leu Glu
   1670 1675 1680
   Phe Lys Arg Leu Ala Asn Ser Lys Ala His Thr Ser Arg Phe Ile
   1685 1690 1695
   Ser Ala Asn Leu Pro Cys Asn Lys Phe Lys Asn Arg Leu Val Asn
   1700 1705 1710
   Ile Met Pro Tyr Glu Ser Thr Arg Val Cys Leu Gln Pro Ile Arg
   1715 1720 1725
   Gly Val Glu Gly Ser Asp Tyr Ile Asn Ala Ser Phe Ile Asp Gly
   1730 1735 1740
   Tyr Arg Gln Gln Lys Ala Tyr Ile Ala Thr Gln Gly Pro Leu Ala
   1745 1750 1755
   Glu Thr Thr Glu Asp Phe Trp Arg Met Leu Trp Glu Asn Asn Ser
   1760 1765 1770
   Thr Ile Val Val Met Leu Thr Lys Leu Arg Glu Met Gly Arg Glu
   1775 1780 1785
   Lys Cys His Gln Tyr Trp Pro Ala Glu Arg Ser Ala Arg Tyr Gln
   1790 1795 1800
   Tyr Phe Val Val Asp Pro Met Ala Glu Tyr Asn Met Pro Gln Tyr
   1805 1810 1815
   Ile Leu Arg Glu Phe Lys Val Thr Asp Ala Arg Asp Gly Gln Ser
   1820 1825 1830
   Arg Thr Val Arg Gln Phe Gln Phe Thr Asp Trp Pro Glu Gln Gly
   1835 1840 1845
   Val Pro Lys Ser Gly Glu Gly Phe Ile Asp Phe Ile Gly Gln Val
   1850 1855 1860
   His Lys Thr Lys Glu Gln Phe Gly Gln Asp Gly Pro Ile Ser Val
   1865 1870 1875
   His Cys Ser Ala Gly Val Gly Arg Thr Gly Val Phe Ile Thr Leu
   1880 1885 1890
   Ser Ile Val Leu Glu Arg Met Arg Tyr Glu Gly Val Val Asp Ile
   1895 1900 1905
   Phe Gln Thr Val Lys Met Leu Arg Thr Gln Arg Pro Ala Met Val
   1910 1915 1920
   Gln Thr Glu Asp Glu Tyr Gln Phe Cys Tyr Gln Ala Ala Leu Glu
   1925 1930 1935
   Tyr Leu Gly Ser Phe Asp His Tyr Ala Thr
   1940 1945
<210> 9
   <211> 1228
   <212> PRT
   <213> Mus musculus
<400> 9
   Glu Glu Pro Pro Arg Phe Ile Arg Glu Pro Lys Asp Gln Ile Gly Val
   1 5 10 15
   Ser Gly Gly Val Ala Ser Phe Val Cys Gln Ala Thr Gly Asp Pro Lys
   20 25 30
   Pro Arg Val Thr Trp Asn Lys Lys Gly Lys Lys Val Asn Ser Gln Arg
   35 40 45
   Phe Glu Thr Ile Asp Phe Asp Glu Ser Ser Gly Ala Val Leu Arg Ile
       50                  55                  60
   Gln Pro Leu Arg Thr Pro Arg Asp Glu Asn Val Tyr Glu Cys Val Ala
   65                  70                  75                  80
   Gln Asn Ser Val Gly Glu Ile Thr Ile His Ala Lys Leu Thr Val Leu
                   85                  90                  95
   Arg Glu Asp Gln Leu Pro Pro Gly Phe Pro Asn Ile Asp Met Gly Pro
               100                 105                 110
   Gln Leu Lys Val Val Glu Arg Thr Arg Thr Ala Thr Met Leu Cys Ala
           115                 120                 125
   Ala Ser Gly Asn Pro Asp Pro Glu Ile Thr Trp Phe Lys Asp Phe Leu
       130                 135                 140
   Pro Val Asp Pro Ser Ala Ser Asn Gly Arg Ile Lys Gln Leu Arg Ser
   145                 150                 155                 160
   Gly Ala Leu Gln Ile Glu Ser Ser Glu Glu Thr Asp Gln Gly Lys Tyr
                   165                 170                 175
   Glu Cys Val Ala Thr Asn Ser Ala Gly Val Arg Tyr Ser Ser Pro Ala
               180                 185                 190
   Asn Leu Tyr Val Arg Val Arg Arg Val Ala Pro Arg Phe Ser Ile Leu
           195                 200                 205
   Pro Met Ser His Glu Ile Met Pro Gly Gly Asn Val Asn Ile Thr Cys
       210                 215                 220
   Val Ala Val Gly Ser Pro Met Pro Tyr Val Lys Trp Met Gln Gly Ala
   225                 230                 235                 240
   Glu Asp Leu Thr Pro Glu Asp Asp Met Pro Val Gly Arg Asn Val Leu
                   245                 250                 255
   Glu Leu Thr Asp Val Lys Asp Ser Ala Asn Tyr Thr Cys Val Ala Met
               260                 265                 270
   Ser Ser Leu Gly Val Ile Glu Ala Val Ala Gln Ile Thr Val Lys Ser
           275                 280                 285
   Leu Pro Lys Ala Pro Gly Thr Pro Val Val Thr Glu Asn Thr Ala Thr
       290                 295                 300
   Ser Ile Thr Val Thr Trp Asp Ser Gly Asn Pro Asp Pro Val Ser Tyr
   305                 310                 315                 320
   Tyr Val Ile Glu Tyr Lys Ser Lys Ser Gln Asp Gly Pro Tyr Gln Ile
                   325                 330                 335
   Lys Glu Asp Ile Thr Thr Thr Arg Tyr Ser Ile Gly Gly Leu Ser Pro
               340                 345                 350
   Asn Ser Glu Tyr Glu Ile Trp Val Ser Ala Val Asn Ser Ile Gly Gln
           355                 360                 365
   Gly Pro Pro Ser Glu Ser Val Val Thr Arg Thr Gly Glu Gln Ala Pro
       370                 375                 380
   Ala Ser Ala Pro Arg Asn Val Gln Ala Arg Met Leu Ser Ala Thr Thr
   385                 390                 395                 400
   Met Ile Val Gln Trp Glu Glu Pro Val Glu Pro Asn Gly Leu Ile Arg
                   405                 410                 415
   Gly Tyr Arg Val Tyr Tyr Thr Met Glu Pro Glu His Pro Val Gly Asn
               420                 425                 430
   Trp Gln Lys His Asn Val Asp Asp Ser Leu Leu Thr Thr Val Gly Ser
           435                 440                 445
   Leu Leu Glu Asp Glu Thr Tyr Thr Val Arg Val Leu Ala Phe Thr Ser
       450                 455                 460
   Val Gly Asp Gly Pro Leu Ser Asp Pro Ile Gln Val Lys Thr Gln Gln
   465                 470                 475                 480
   Gly Val Pro Gly Gln Pro Met Asn Leu Arg Ala Glu Ala Lys Ser Glu
                   485                 490                 495
   Thr Ser Ile Gly Leu Ser Trp Ser Ala Pro Arg Gln Glu Ser Val Ile
               500                 505                 510
   Lys Tyr Glu Leu Leu Phe Arg Glu Gly Asp Arg Gly Arg Glu Val Gly
           515                 520                 525
   Arg Thr Phe Asp Pro Thr Thr Ala Phe Val Val Glu Asp Leu Lys Pro
       530                 535                 540
   Asn Thr Glu Tyr Ala Phe Arg Leu Ala Ala Arg Ser Pro Gln Gly Leu
   545 550 555 560
   Gly Ala Phe Thr Ala Val Val Arg Gln Arg Thr Leu Gln Ala Lys Pro
   565 570 575
   Ser Ala Pro Pro Gln Asp Val Lys Cys Thr Ser Leu Arg Ser Thr Ala
   580 585 590
   Ile Leu Val Ser Trp Arg Pro Pro Pro Pro Glu Thr His Asn Gly Ala
   595 600 605
   Leu Val Gly Tyr Ser Val Arg Tyr Arg Pro Leu Gly Ser Glu Asp Pro
   610 615 620
   Asp Pro Lys Glu Val Asn Asn Ile Pro Pro Thr Thr Thr Gln Ile Leu
   625 630 635 640
   Leu Glu Ala Leu Glu Lys Trp Thr Glu Tyr Arg Val Thr Ala Val Ala
   645 650 655
   Tyr Thr Glu Val Gly Pro Gly Pro Glu Ser Ser Pro Val Val Val Arg
   660 665 670
   Thr Asp Glu Asp Val Pro Ser Ala Pro Pro Arg Lys Val Glu Ala Glu
   675 680 685
   Ala Leu Asn Ala Thr Ala Ile Arg Val Leu Trp Arg Ser Pro Thr Pro
   690 695 700
   Gly Arg Gln His Gly Gln Ile Arg Gly Tyr Gln Val His Tyr Val Arg
   705 710 715 720
   Met Glu Gly Ala Glu Ala Arg Gly Pro Pro Arg Ile Lys Asp Ile Met
   725 730 735
   Leu Ala Asp Ala Gln Glu Met Val Ile Thr Asn Leu Gln Pro Glu Thr
   740 745 750
   Ala Tyr Ser Ile Thr Val Ala Ala Tyr Thr Met Lys Gly Asp Gly Ala
   755 760 765
   Arg Ser Lys Pro Lys Val Val Val Thr Lys Gly Ala Val Leu Gly Arg
   770 775 780
   Pro Thr Leu Ser Val Gln Gln Thr Pro Glu Gly Ser Leu Leu Ala Arg
   785 790 795 800
   Trp Glu Pro Pro Ala Asp Ala Ala Glu Asp Pro Val Leu Gly Tyr Arg
                   805                 810                 815
   Leu Gln Phe Gly Arg Glu Asp Ala Ala Pro Ala Thr Leu Glu Leu Ala
               820                 825                 830
   Ala Trp Glu Arg Arg Phe Ala Ala Pro Ala His Lys Gly Ala Thr Tyr
           835                 840                 845
   Val Phe Arg Leu Ala Ala Arg Gly Arg Ala Gly Leu Gly Glu Glu Ala
       850                 855                 860
   Ala Ala Ala Leu Ser Ile Pro Glu Asp Ala Pro Arg Gly Phe Pro Gln
   865                 870                 875                 880
   Ile Leu Gly Ala Ala Gly Asn Val Ser Ala Gly Ser Val Leu Leu Arg
                   885                 890                 895
   Trp Leu Pro Pro Val Pro Ala Glu Arg Asn Gly Ala Ile Ile Lys Tyr
               900                 905                 910
   Thr Val Ser Val Arg Glu Ala Gly Ala Pro Gly Pro Ala Thr Glu Thr
           915                 920                 925
   Glu Leu Ala Ala Ala Ala Gln Pro Gly Ala Glu Thr Ala Leu Thr Leu
       930                 935                 940
   Arg Gly Leu Arg Pro Glu Thr Ala Tyr Glu Leu Arg Val Arg Ala His
   945                 950                 955                 960
   Thr Arg Arg Gly Pro Gly Pro Phe Ser Pro Pro Leu Arg Tyr Arg Leu
                   965                 970                 975
   Ala Arg Asp Pro Val Ser Pro Lys Asn Phe Lys Val Lys Met Ile Met
               980                 985                 990
   Lys Thr Ser Val Leu Leu Ser Trp  Glu Phe Pro Asp Asn  Tyr Asn Ser
           995                 1000                1005
   Pro Thr  Pro Tyr Lys Ile Gln  Tyr Asn Gly Leu Thr  Leu Asp Val
       1010                1015                1020
   Asp Gly  Arg Thr Thr Lys Lys  Leu Ile Thr His Leu  Lys Pro His
       1025                1030                1035
   Thr Phe  Tyr Asn Phe Val Leu  Thr Asn Arg Gly Ser  Ser Leu Gly
       1040                1045                1050
   Gly Leu Gln Gln Thr Val Thr Ala Arg Thr Ala Phe Asn Met Leu
   1055 1060 1065
   Ser Gly Lys Pro Ser Val Ala Pro Lys Pro Asp Asn Asp Gly Phe
   1070 1075 1080
   Ile Val Val Tyr Leu Pro Asp Gly Gln Ser Pro Val Thr Val Gln
   1085 1090 1095
   Asn Tyr Phe Ile Val Met Val Pro Leu Arg Lys Ser Arg Gly Gly
   1100 1105 1110
   Gln Phe Pro Val Leu Leu Gly Ser Pro Glu Asp Met Asp Leu Glu
   1115 1120 1125
   Glu Leu Ile Gln Asp Ile Ser Arg Leu Gln Arg Arg Ser Leu Arg
   1130 1135 1140
   His Ser Arg Gln Leu Glu Val Pro Arg Pro Tyr Ile Ala Ala Arg
   1145 1150 1155
   Phe Ser Ile Leu Pro Ala Val Phe His Pro Gly Asn Gln Lys Gln
   1160 1165 1170
   Tyr Gly Gly Phe Asp Asn Arg Gly Leu Glu Pro Gly His Arg Tyr
   1175 1180 1185
   Val Leu Phe Val Leu Ala Val Leu Gln Lys Asn Glu Pro Thr Phe
   1190 1195 1200
   Ala Ala Ser Pro Phe Ser Asp Pro Phe Gln Leu Asp Asn Pro Asp
   1205 1210 1215
   Pro Gln Pro Ile Val Asp Gly Glu Glu Gly
   1220 1225
<210> 10
   <211> 1253
   <212> PRT
   <213> Homo sapiens
<400> 10
   Glu Glu Pro Pro Arg Phe Ile Lys Glu Pro Lys Asp Gln Ile Gly Val
   1 5 10 15
   Ser Gly Gly Val Ala Ser Phe Val Cys Gln Ala Thr Gly Asp Pro Lys
   20 25 30
   Pro Arg Val Thr Trp Asn Lys Lys Gly Lys Lys Val Asn Ser Gln Arg
           35                  40                  45
   Phe Glu Thr Ile Glu Phe Asp Glu Ser Ala Gly Ala Val Leu Arg Ile
       50                  55                  60
   Gln Pro Leu Arg Thr Pro Arg Asp Glu Asn Val Tyr Glu Cys Val Ala
   65                  70                  75                  80
   Gln Asn Ser Val Gly Glu Ile Thr Val His Ala Lys Leu Thr Val Leu
                   85                  90                  95
   Arg Glu Asp Gln Leu Pro Ser Gly Phe Pro Asn Ile Asp Met Gly Pro
               100                 105                 110
   Gln Leu Lys Val Val Glu Arg Thr Arg Thr Ala Thr Met Leu Cys Ala
           115                 120                 125
   Ala Ser Gly Asn Pro Asp Pro Glu Ile Thr Trp Phe Lys Asp Phe Leu
       130                 135                 140
   Pro Val Asp Pro Ser Ala Ser Asn Gly Arg Ile Lys Gln Leu Arg Ser
   145                 150                 155                 160
   Glu Thr Phe Glu Ser Thr Pro Ile Arg Gly Ala Leu Gln Ile Glu Ser
                   165                 170                 175
   Ser Glu Glu Thr Asp Gln Gly Lys Tyr Glu Cys Val Ala Thr Asn Ser
               180                 185                 190
   Ala Gly Val Arg Tyr Ser Ser Pro Ala Asn Leu Tyr Val Arg Glu Leu
           195                 200                 205
   Arg Glu Val Arg Arg Val Ala Pro Arg Phe Ser Ile Leu Pro Met Ser
       210                 215                 220
   His Glu Ile Met Pro Gly Gly Asn Val Asn Ile Thr Cys Val Ala Val
   225                 230                 235                 240
   Gly Ser Pro Met Pro Tyr Val Lys Trp Met Gln Gly Ala Glu Asp Leu
                   245                 250                 255
   Thr Pro Glu Asp Asp Met Pro Val Gly Arg Asn Val Leu Glu Leu Thr
               260                 265                 270
   Asp Val Lys Asp Ser Ala Asn Tyr Thr Cys Val Ala Met Ser Ser Leu
           275                 280                 285
   Gly Val Ile Glu Ala Val Ala Gln Ile Thr Val Lys Ser Leu Pro Lys
       290                 295                 300
   Ala Pro Gly Thr Pro Met Val Thr Glu Asn Thr Ala Thr Ser Ile Thr
   305                 310                 315                 320
   Ile Thr Trp Asp Ser Gly Asn Pro Asp Pro Val Ser Tyr Tyr Val Ile
                   325                 330                 335
   Glu Tyr Lys Ser Lys Ser Gln Asp Gly Pro Tyr Gln Ile Lys Glu Asp
               340                 345                 350
   Ile Thr Thr Thr Arg Tyr Ser Ile Gly Gly Leu Ser Pro Asn Ser Glu
           355                 360                 365
   Tyr Glu Ile Trp Val Ser Ala Val Asn Ser Ile Gly Gln Gly Pro Pro
       370                 375                 380
   Ser Glu Ser Val Val Thr Arg Thr Gly Glu Gln Ala Pro Ala Ser Ala
   385                 390                 395                 400
   Pro Arg Asn Val Gln Ala Arg Met Leu Ser Ala Thr Thr Met Ile Val
                   405                 410                 415
   Gln Trp Glu Glu Pro Val Glu Pro Asn Gly Leu Ile Arg Gly Tyr Arg
               420                 425                 430
   Val Tyr Tyr Thr Met Glu Pro Glu His Pro Val Gly Asn Trp Gln Lys
           435                 440                 445
   His Asn Val Asp Asp Ser Leu Leu Thr Thr Val Gly Ser Leu Leu Glu
       450                 455                 460
   Asp Glu Thr Tyr Thr Val Arg Val Leu Ala Phe Thr Ser Val Gly Asp
   465                 470                 475                 480
   Gly Pro Leu Ser Asp Pro Ile Gln Val Lys Thr Gln Gln Gly Val Pro
                   485                 490                 495
   Gly Gln Pro Met Asn Leu Arg Ala Glu Ala Arg Ser Glu Thr Ser Ile
               500                 505                 510
   Thr Leu Ser Trp Ser Pro Pro Arg Gln Glu Ser Ile Ile Lys Tyr Glu
           515                 520                 525
   Leu Leu Phe Arg Glu Gly Asp His Gly Arg Glu Val Gly Arg Thr Phe
       530                 535                 540
   Asp Pro Thr Thr Ser Tyr Val Val Glu Asp Leu Lys Pro Asn Thr Glu
   545                 550                 555                 560
   Tyr Ala Phe Arg Leu Ala Ala Arg Ser Pro Gln Gly Leu Gly Ala Phe
                   565                 570                 575
   Thr Pro Val Val Arg Gln Arg Thr Leu Gln Ser Lys Pro Ser Ala Pro
               580                 585                 590
   Pro Gln Asp Val Lys Cys Val Ser Val Arg Ser Thr Ala Ile Leu Val
           595                 600                 605
   Ser Trp Arg Pro Pro Pro Pro Glu Thr His Asn Gly Ala Leu Val Gly
       610                 615                 620
   Tyr Ser Val Arg Tyr Arg Pro Leu Gly Ser Glu Asp Pro Glu Pro Lys
   625                 630                 635                 640
   Glu Val Asn Gly Ile Pro Pro Thr Thr Thr Gln Ile Leu Leu Glu Ala
                   645                 650                 655
   Leu Glu Lys Trp Thr Gln Tyr Arg Ile Thr Thr Val Ala His Thr Glu
               660                 665                 670
   Val Gly Pro Gly Pro Glu Ser Ser Pro Val Val Val Arg Thr Asp Glu
           675                 680                 685
   Asp Val Pro Ser Ala Pro Pro Arg Lys Val Glu Ala Glu Ala Leu Asn
       690                 695                 700
   Ala Thr Ala Ile Arg Val Leu Trp Arg Ser Pro Ala Pro Gly Arg Gln
   705                 710                 715                 720
   His Gly Gln Ile Arg Gly Tyr Gln Val His Tyr Val Arg Met Glu Gly
                   725                 730                 735
   Ala Glu Ala Arg Gly Pro Pro Arg Ile Lys Asp Val Met Leu Ala Asp
               740                 745                 750
   Ala Gln Trp Glu Thr Asp Asp Thr Ala Glu Tyr Glu Met Val Ile Thr
           755                 760                 765
   Asn Leu Gln Pro Glu Thr Ala Tyr Ser Ile Thr Val Ala Ala Tyr Thr
       770                 775                 780
   Met Lys Gly Asp Gly Ala Arg Ser Lys Pro Lys Val Val Val Thr Lys
   785                 790                 795                 800
   Gly Ala Val Leu Gly Arg Pro Thr Leu Ser Val Gln Gln Thr Pro Glu
                   805                 810                 815
   Gly Ser Leu Leu Ala Arg Trp Glu Pro Pro Ala Gly Thr Ala Glu Asp
               820                 825                 830
   Gln Val Leu Gly Tyr Arg Leu Gln Phe Gly Arg Glu Asp Ser Thr Pro
           835                 840                 845
   Leu Ala Thr Leu Glu Phe Pro Pro Ser Glu Asp Arg Tyr Thr Ala Ser
       850                 855                 860
   Gly Val His Lys Gly Ala Thr Tyr Val Phe Arg Leu Ala Ala Arg Ser
   865                 870                 875                 880
   Arg Gly Gly Leu Gly Glu Glu Ala Ala Glu Val Leu Ser Ile Pro Glu
                   885                 890                 895
   Asp Thr Pro Arg Gly His Pro Gln Ile Leu Glu Ala Ala Gly Asn Ala
               900                 905                 910
   Ser Ala Gly Thr Val Leu Leu Arg Trp Leu Pro Pro Val Pro Ala Glu
           915                 920                 925
   Arg Asn Gly Ala Ile Val Lys Tyr Thr Val Ala Val Arg Glu Ala Gly
       930                 935                 940
   Ala Leu Gly Pro Ala Arg Glu Thr Glu Leu Pro Ala Ala Ala Glu Pro
   945                 950                 955                 960
   Gly Ala Glu Asn Ala Leu Thr Leu Gln Gly Leu Lys Pro Asp Thr Ala
                   965                 970                 975
   Tyr Asp Leu Gln Val Arg Ala His Thr Arg Arg Gly Pro Gly Pro Phe
               980                 985                 990
   Ser Pro Pro Val Arg Tyr Arg Thr  Phe Leu Arg Asp Gln  Val Ser Pro
           995                 1000                 1005
   Lys Asn  Phe Lys Val Lys Met  Ile Met Lys Thr Ser  Val Leu Leu
       1010                 1015                 1020
   Ser Trp  Glu Phe Pro Asp Asn  Tyr Asn Ser Pro Thr  Pro Tyr Lys
       1025                 1030                 1035
   Ile Gln Tyr Asn Gly Leu Thr Leu Asp Val Asp Gly Arg Thr Thr
       1040                1045                1050
   Lys Lys Leu Ile Thr His Leu Lys Pro His Thr Phe Tyr Asn Phe
       1055                1060                1065
   Val Leu Thr Asn Arg Gly Ser Ser Leu Gly Gly Leu Gln Gln Thr
       1070                1075                1080
   Val Thr Ala Trp Thr Ala Phe Asn Leu Leu Asn Gly Lys Pro Ser
       1085                1090                1095
   Val Ala Pro Lys Pro Asp Ala Asp Gly Phe Ile Met Val Tyr Leu
       1100                1105                1110
   Pro Asp Gly Gln Ser Pro Val Pro Val Gln Ser Tyr Phe Ile Val
       1115                1120                1125
   Met Val Pro Leu Arg Lys Ser Arg Gly Gly Gln Phe Leu Thr Pro
       1130                1135                1140
   Leu Gly Ser Pro Glu Asp Met Asp Leu Glu Glu Leu Ile Gln Asp
       1145                1150                1155
   Ile Ser Arg Leu Gln Arg Arg Ser Leu Arg His Ser Arg Gln Leu
       1160                1165                1170
   Glu Val Pro Arg Pro Tyr Ile Ala Ala Arg Phe Ser Val Leu Pro
       1175                1180                1185
   Pro Thr Phe His Pro Gly Asp Gln Lys Gln Tyr Gly Gly Phe Asp
       1190                1195                1200
   Asn Arg Gly Leu Glu Pro Gly His Arg Tyr Val Leu Phe Val Leu
       1205                1210                1215
   Ala Val Leu Gln Lys Ser Glu Pro Thr Phe Ala Ala Ser Pro Phe
       1220                1225                1230
   Ser Asp Pro Phe Gln Leu Asp Asn Pro Asp Pro Gln Pro Ile Val
       1235                1240                1245
   Asp Gly Glu Glu Gly
       1250
<210> 11
   <211> 86
   <212> PRT
   <213> Artificial Sequence
<220>
   <223> Synthetic polypeptide
<400> 11
   Pro Lys Asp Gln Ile Gly Val Ser Gly Gly Val Ala Ser Phe Val Cys
   1               5                   10                  15
   Gln Ala Thr Gly Asp Pro Lys Pro Arg Val Thr Trp Asn Lys Lys Gly
               20                  25                  30
   Lys Lys Val Asn Ser Gln Arg Phe Glu Thr Ile Glu Phe Asp Glu Ser
           35                  40                  45
   Ala Gly Ala Val Leu Arg Ile Gln Pro Leu Arg Thr Pro Arg Asp Glu
       50                  55                  60
   Asn Val Tyr Glu Cys Val Ala Gln Asn Ser Val Gly Glu Ile Thr Val
   65                  70                  75                  80
   His Ala Lys Leu Thr Val
                   85
<210> 12
   <211> 82
   <212> PRT
   <213> Artificial Sequence
<220>
   <223> Synthetic polypeptide
<400> 12
   Ala Thr Met Leu Cys Ala Ala Ser Gly Asn Pro Asp Pro Glu Ile Thr
   1               5                   10                  15
   Trp Phe Lys Asp Phe Leu Pro Val Asp Pro Ser Ala Ser Asn Gly Arg
               20                  25                  30
   Ile Lys Gln Leu Arg Ser Glu Thr Phe Glu Ser Thr Pro Ile Arg Gly
           35                  40                  45
   Ala Leu Gln Ile Glu Ser Ser Glu Glu Thr Asp Gln Gly Lys Tyr Glu
       50                  55                  60
   Cys Val Ala Thr Asn Ser Ala Gly Val Arg Tyr Ser Ser Pro Ala Asn
   65                  70                  75                  80
   Leu Tyr
<210> 13
   <211> 69
   <212> PRT
   <213> Artificial Sequence
<220>
   <223> Synthetic polypeptide
<400> 13
   Gly Gly Asn Val Asn Ile Thr Cys Val Ala Val Gly Ser Pro Met Pro
   1               5                   10                  15
   Tyr Val Lys Trp Met Gln Gly Ala Glu Asp Leu Thr Pro Glu Asp Asp
               20                  25                  30
   Met Pro Val Gly Arg Asn Val Leu Glu Leu Thr Asp Val Lys Asp Ser
           35                  40                  45
   Ala Asn Tyr Thr Cys Val Ala Met Ser Ser Leu Gly Val Ile Glu Ala
       50                  55                  60
   Val Ala Gln Ile Thr
   65

## Description

### BACKGROUND OF THE INVENTION

Approximately 5 to 8% of people in the United States suffer from an autoimmune disease. Researchers have identified more than 80 different autoimmune diseases and suspect that many more diseases may have an autoimmune component. Rheumatoid arthritis (RA) alone afflicts roughly 2.5 million people in the United States RA affects the joints and bones but may also affect different organs and biological systems.

Fibroblast-like synoviocytes (FLS) are key players in mediating inflammation and joint destruction in rheumatoid arthritis (RA). There is an increased level of attention to this cell type as the possible target of a new generation of anti-RA therapies, which would be used in combination with immunomodulators to help control disease without increasing immune-suppression. The behavior of FLS is controlled by multiple interconnected signal transduction pathways. Several of these pathways involve reversible phosphorylation of proteins on tyrosine residues, which is the result of the balanced action of protein tyrosine kinases (PTKs) and phosphatases (PTPs). PTKs are mediators of FLS growth and invasiveness. PTPs act by removing phosphate groups from phosphorylated tyrosine residues on proteins. Receptor protein tyrosine phosphatases (RPTPs or PTPRs) are PTPs that generally have a variable length extracellular domain followed by a transmembrane region and a C-terminal catalytic cytoplasmic domain. However, little is known regarding the connection between PTPs or PTPRs in relation to FLS. In WO2012/148003 anti-PTPRS antibodies are provided that can detect, identify or isolate plasmacytoid dendritic cells (pDC). Coles et al., Science (2011), Vol. 332, 484-488 describes crystallographic studies to investigate the structural basis of proteoglycan recognition in RPTPs and type IIa RPTPs in general. Provided herein are methods and compositions addressing these and other needs in the art.

### BRIEF SUMMARY OF THE INVENTION

The invention is defined in the claims. The invention provides a non-enzymatic recombinant protein comprising an amino acid sequence of an extracellular domain of PTPRS, wherein the protein comprises both PTPRS immunoglobulin-like domain 1 (Ig1) and immunoglobulin-like domain 2 (Ig2), for use in therapy.

The invention also provides a PTPRS de-clustering agent for use in a method of treating arthritis wherein the PTPRS de-clustering agent is selected from: the non-enzymatic recombinant protein as defined herein; an anti-PTPRS antibody or fragment thereof; or an anti-heparan sulfate antibody, the method comprising administering to the subject a therapeutically effective amount of the PTPRS de-clustering agent, wherein administration treats arthritis in the subject.

Described herein are methods of modulating extracellular matrix in a subject. The methods include administering to the subject an effective amount of a non-enzymatic recombinant protein comprising an amino acid sequence of an extracellular domain of PTPRS or portion thereof.

Described herein are methods of identifying a candidate PTPRS de-clustering agent. The methods include contacting a test agent with clustered PTPRS proteins and detecting de-clustering of the PTPRS proteins, thereby identifying a candidate PTPRS de-clustering agent. Alternatively, the methods include contacting a test agent with PTPRS and heparan sulfate and determining whether the test agent inhibits binding of the PTPRS to heparan sulfate, inhibition of binding indicating the test agent is a PTPRS de-clustering agent.

### BRIEF DESCRIPTION OF THE DRAWINGS

Figures 1A and 1B are graphs depicting expression of PTPRS in fibroblast-like synoviocytes (FLS). Figure 1A shows expression in mice. Figure 1B shows expression in humans.

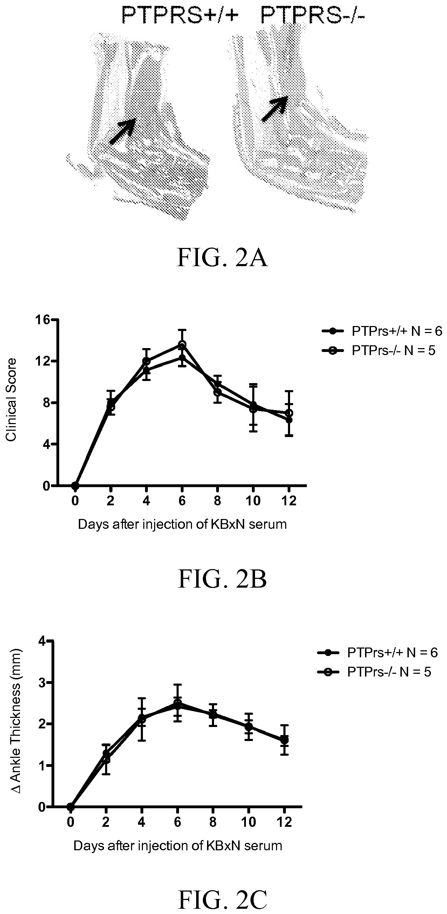

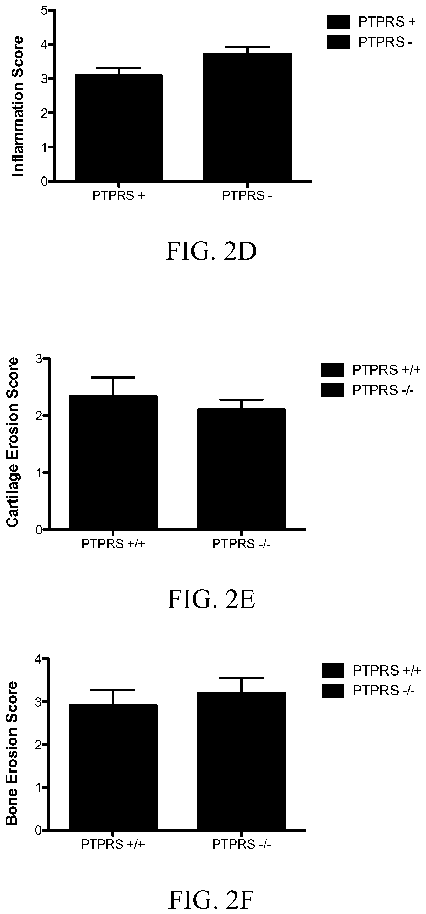

Figures 2A, 2B, 2C, 2D, 2E, and 2F are picture and graphs depicting increased bone erosion severity in arthritis for PTPRS -/- mice compared with PTPRS +/+ mice. Mice were injected with 100 µL K/BxN serum intraperitoneally at 8-10 weeks of age (PTPRS-/- received proportionally less serum to reflect their decreased body weight). Ankle thickness was measured with digitals calipers and ankles and wrists were given a clinical arthritis score between 0 (no redness or swelling) and 4 (maximal swelling with digits involved) every two days for 14 days. See Figures 2A, 2B, and 2C. After 14 days, mice were euthanized and ankles fixed for histological scoring using H&E (inflammation and bone erosion) and safranin O (cartliage erosion) staining. See Figures 2D (inflammation), 2E (cartilage erosion), and 2F (bone erosion) for histology scores.
Figure 3 is a histogram showing that treatment of mouse FLS (mFLS) with chondroitin sulfate (CS) reduces their invasion through an extracellular matrix. PTPRS-/- mFLS exhibit reduced CS-mediated inhibition of invasion.
Figure 4 is a graph showing invasiveness of PTPRS-/- mFLS is unaffected by CS at levels approximately 5 times higher than those in the joint.
Figure 5 is a graph showing that treatment of mFLS with CS reduces their migration. PTPRS-/- mFLS exhibit reduced CS-mediated inhibition of migration.
Figure 6 are images of gels showing vivo-morpholino mediated knockdown of PTPRS in mouse and human FLS. Left panel: murine MLS: right panel: human FLS.
Figure 7 is a histogram showing PTPRS knockdown increases migration of human RA FLS and abrogates CS-mediated inhibition of migration.
Figure 8 are graphs showing mRNA PTPome analysis of RA FLS shows that at least 5 PTPs are highly expressed.
Figures 9A and 9B are graphs showing expression of PTPRS mRNA. Expression in Figure 9A FLS sorted from control mice or mice with K/BxN serum induced acute or chronic arthritis and Figure 9B mouse sorted macrophages and FLS with PTPRC included as a positive control for expression in macrophages. PTP expression in FLS and macrophages from pooled mice was measured by quantitative polymerase chain reaction. Values are the mean of PTP expression relative to the housekeeping gene GAPDH.

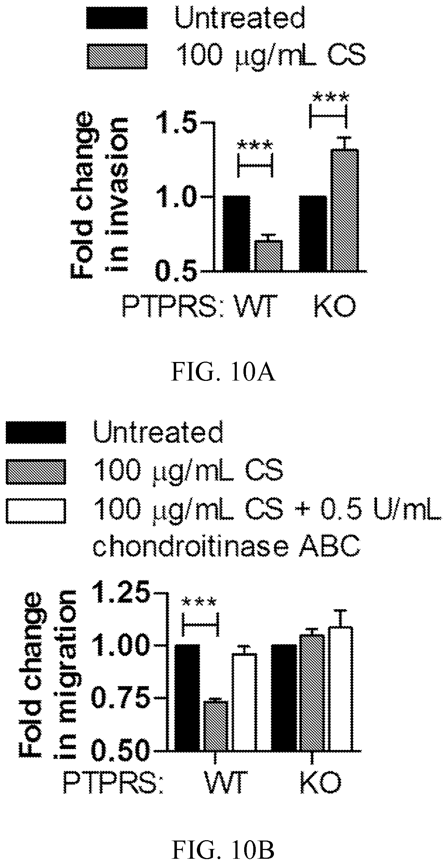

Figures 10A and 10B are graphs showing FLS migration. In Figure 10A, PTPRS WT or KO FLS were allowed to invade through Matrigel-coated transwell chambers. Cells per field were counted after invasion for 24 hours in response to 5% fetal bovine serum (FBS) in the presence of CS. In Figure 10B, PTPRS WT or KO FLS were allowed to migrate through uncoated transwell chambers. Cells per field were counted after migration for 4 h in response to 5% FBS in the presence of CS with or without chondroitinase ABC treatment. Bars indicate mean +/- standard error of the fold-change of migration relative to that of untreated cells. Data include three independent experiments using three sets of littermate cell lines. (***P < 0.001; significance determined using Mann-Whitney test on raw data).

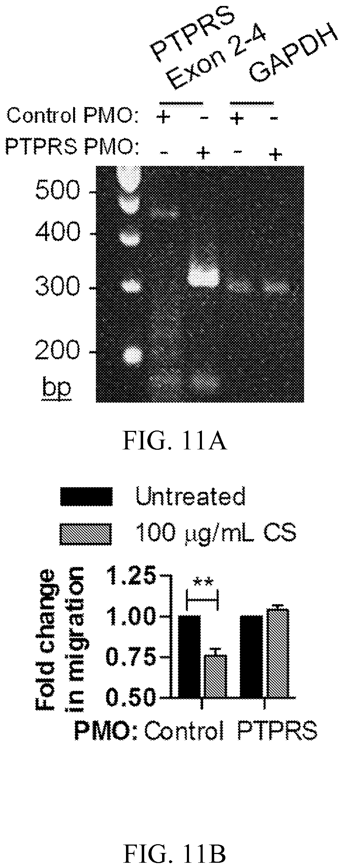

Figures 11A and 11B are graphs showing inhibition of RA FLS migration by chondroitin sulfate (CS) is dependent on PTPRS expression. Figure 11A is a graph of RT-PCR data showing knock-down of PTPRS in RA FLS after incubation with anti-sense oligo (ASO) for 7 days. In Figure 11B, PTPRS WT or KO FLS were allowed to migrate through uncoated transwell chambers. Cells per field were counted after migration for 4 h in response to 5% fetal bovine serum (FBS) in the presence of CS with or without chondroitinase ABC treatment. Bars indicate mean +/- standard error of the fold-change of migration relative to that of untreated cells. Data include three independent experiments using three sets of littermate cell lines. (***P < 0.001; significance determined using Mann-Whitney test on raw data).

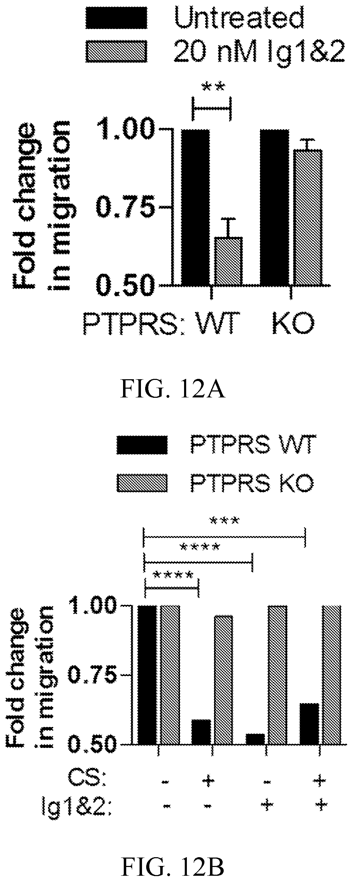

Figures 12A and 12B are graphs showing displacement of PTPRS from HS is sufficient to inhibit FLS migration and invasion. PTPRS WT or KO FLS were allowed to invade through uncoated transwell chambers. Cells per field were counted after migration for 4 h in response to 5% fetal bovine serum (FBS) in the presence of 20 nM of the Ig1 and Ig2 domains of PTPRS (Ig1+2) alone (Figure 12A) or in addition to 100 µg/mL CS (Figure 12B). Bars indicate mean +/standard error of the fold-change of migration relative to that of untreated cells. Data include three independent experiments using three sets of littermate cell lines. (**P < 0.01, ***P < 0.001, ****P < 0.0001; significance determined using Mann-Whitney test on raw data).
Figures 13A and 13B are pictures of light micrographs of bovine cartilage explants and a graph. Figure 13A shows light micrographs stained with Mayer's hematoxylin with and without Ig1+2 treatment. Figure 13B is a graph showing cell counts with and without Ig1+2 treatment.
Figures 14A and 14B are graphs showing beta-catenin and cadherin binding and desphosphorylation. Figure 14A is a Western blot of total cell lysates or lysates incubated with GST or GST-PTPRS D/A substrate trapping mutant from unstimulated or pervanadate stimulated FLS. * represents GST-PTPRS D1 D/A and ** represents GST tag. Figure 14B Western blot showing decreased tyrosine phosphorylation of immunopurified beta-catenin in the presence of GST-PTPRS D1 compared to GST alone.
Figure 15 is a graph showing Ig1 and Ig2 effect in ameliorating arthritis. Clinical Score of BALB/c mice induced with acute arthritis by administration of 100 µL K/BxN serum on Day 0 and treated with vehicle (control) or 500 µg Ig1+2 daily from days 0 to 6. N=3 per treatment. Histopathological analysis of the joint with (lower panel) and without (upper panel) Ig1+2 treatment plus erosion and cartilage damage score.
Figure 16 is a graph showing treatment of mFLS with recombinant PTPRS Ig1+2 reduces migration.
Figure 17 is a graph showing PTPRS-/- mFLS are insensitive to recombinant PTPRS Ig1+2 mediated reduced migration.
Figure 18 is a schematic of proposed PTPRS, chondroitin sulfate and heparin sulfate activity in fibroblast-like synoviocytes in the joint.

### DETAILED DESCRIPTION OF THE INVENTION

The present disclosure relates to the transmembrane PTP commonly referred to as Protein Tyrosine Phosphatase, Receptor Type, S or Sigma (PTPRS). The extracellular domain of PTPRS includes multiple fibronectin type III-like domains and immunoglobulin-like (Ig) domains. The two most external Ig domains (called Ig1 and Ig2) interact with nanomolar affinity with HS- (heparan sulfate) and CS- (chondroitin sulfate) containing PG (proteoglycan). The interaction of PTPRS with HS (which is layered on the cell surface of most cell types) induces a clustered topology of PTPRS, which inhibits the phosphatase action and promotes signaling. On the contrary, CS (enriched in the ECM surrounding neurons and other cells) induces a declustered conformation, which is active and leads to signaling inhibition. Fruther information regarding PTPRS may be found, for example, in Shen et al., Science 23 October 2009, 592-596; Coles et al., Science 22 April 2011, 484-488.

As demonstrated herein, the extracellular domain of PTPRS binds to proteoglycans in the extracellular matrix. This binding to different proteoglycans results in differences in the intracellular functions of the phosphatase. This PTPRS-mediated mechanism of regulation of intracellular signaling by the extracellular matrix is referred to herein as "the proteoglycan switch". As demonstrated herein, the proteoglycan switch regulates in a PTPRS-dependent way the adhesion and invasiveness of FLS. Interfering with the proteoglycan switch *in vivo* leads to decreased severity of arthritis in a mouse model. Thus, as demonstrated herein, PTPRS is a key regulator of RA FLS destructive behavior.

The terms "subject," "patient," "individual," etc. are not intended to be limiting and can be generally interchanged. That is, an individual described as a "patient" does not necessarily have a given disease, but may be merely seeking medical advice.

A "control" or "standard control" refers to a sample, measurement, or value that serves as a reference, usually a known reference, for comparison to a test sample, measurement, or value. For example, a test sample can be taken from a patient suspected of having a given disease (e.g. an autoimmune disease, inflammatory autoimmune disease, cancer, infectious disease, immune disease, or other disease) and compared to a known normal (non-diseased) individual (e.g. a standard control subject). A standard control can also represent an average measurement or value gathered from a population of similar individuals (e.g. standard control subjects) that do not have a given disease (i.e. standard control population), e.g., healthy individuals with a similar medical background, same age, weight, etc. A standard control value can also be obtained from the same individual, e.g. from an earlier-obtained sample from the patient prior to disease onset. One of skill will recognize that standard controls can be designed for assessment of any number of parameters (e.g. RNA levels, protein levels, specific cell types, specific bodily fluids, specific tissues, synoviocytes, synovial fluid, synovial tissue, fibroblast-like synoviocytes, macrophagelike synoviocytes, etc).

One of skill in the art will understand which standard controls are most appropriate in a given situation and be able to analyze data based on comparisons to standard control values. Standard controls are also valuable for determining the significance (e.g. statistical significance) of data. For example, if values for a given parameter are widely variant in standard controls, variation in test samples will not be considered as significant.

The terms "dose" and "dosage" are used interchangeably herein. A dose refers to the amount of active ingredient given to an individual at each administration. The dose will vary depending on a number of factors, including the range of normal doses for a given therapy, frequency of administration; size and tolerance of the individual; severity of the condition; risk of side effects; and the route of administration. One of skill will recognize that the dose can be modified depending on the above factors or based on therapeutic progress. The term "dosage form" refers to the particular format of the pharmaceutical or pharmaceutical composition, and depends on the route of administration. For example, a dosage form can be in a liquid form for nebulization, e.g., for inhalants, in a tablet or liquid, e.g., for oral delivery, or a saline solution, e.g., for injection.

As used herein, the terms "treat" and "prevent" may refer to any delay in onset, reduction in the frequency or severity of symptoms, amelioration of symptoms, improvement in patient comfort or function (e.g. joint function), decrease in severity of the disease state, etc. The effect of treatment can be compared to an individual or pool of individuals not receiving a given treatment, or to the same patient prior to, or after cessation of, treatment. The term "prevent" generally refers to a decrease in the occurrence of a given disease (e.g. an autoimmune, inflammatory autoimmune, cancer, infectious, immune, or other disease) or disease symptoms in a patient. As indicated above, the prevention may be complete (no detectable symptoms) or partial, such that fewer symptoms are observed than would likely occur absent treatment.

By "therapeutically effective dose or amount" as used herein is meant a dose that produces effects for which it is administered (e.g. treating or preventing a disease). The exact dose and formulation will depend on the purpose of the treatment, and will be ascertainable by one skilled in the art using known techniques (see, e.g., Lieberman, Pharmaceutical Dosage Forms (vols. 1-3, 1992); Lloyd, The Art, Science and Technology of Pharmaceutical Compounding (1999); Remington: The Science and Practice of Pharmacy, 20th Edition, Gennaro, Editor (2003), and Pickar, Dosage Calculations (1999)). For example, for the given parameter, a therapeutically effective amount will show an increase or decrease of at least 5%, 10%, 15%, 20%, 25%, 40%, 50%, 60%, 75%, 80%, 90%, or at least 100%. Therapeutic efficacy can also be expressed as "-fold" increase or decrease. For example, a therapeutically effective amount can have at least a 1.2-fold, 1.5-fold, 2-fold, 5-fold, or more effect over a standard control. A therapeutically effective dose or amount may ameliorate one or more symptoms of a disease. A therapeutically effective dose or amount may prevent or delay the onset of a disease or one or more symptoms of a disease when the effect for which it is being administered is to treat a person who is at risk of developing the disease.

The term "diagnosis" refers to a relative probability that a disease (e.g. an autoimmune, inflammatory autoimmune, cancer, infectious, immune, or other disease) is present in the subject. Similarly, the term "prognosis" refers to a relative probability that a certain future outcome may occur in the subject with respect to a disease state. For example, in the context of the present invention, prognosis can refer to the likelihood that an individual will develop a disease (e.g. an autoimmune, inflammatory autoimmune, cancer, infectious, immune, or other disease), or the likely severity of the disease (e.g., duration of disease). The terms are not intended to be absolute, as will be appreciated by any one of skill in the field of medical diagnostics.

"Nucleic acid" or "oligonucleotide" or "polynucleotide" or grammatical equivalents used herein means at least two nucleotides covalently linked together. The term "nucleic acid" includes single-, double-, or multiple-stranded DNA, RNA and analogs (derivatives) thereof. Oligonucleotides are typically from about 5, 6, 7, 8, 9, 10, 12, 15, 25, 30, 40, 50 or more nucleotides in length, up to about 100 nucleotides in length. Nucleic acids and polynucleotides are a polymers of any length, including longer lengths, e.g., 200, 300, 500, 1000, 2000, 3000, 5000, 7000, 10,000, etc. The nucleic acids herein may contain phosphodiester bonds. Nucleic acid analogs may have alternate backbones, comprising, e.g., phosphoramidate, phosphorothioate, phosphorodithioate, or O-methylphosphoroamidite linkages (see Eckstein, Oligonucleotides and Analogues: A Practical Approach, Oxford University Press); and peptide nucleic acid backbones and linkages. Other analog nucleic acids include those with positive backbones; non-ionic backbones, and nonribose backbones, including those described in U.S. Patent Nos. 5,235,033 and 5,034,506, and Chapters 6 and 7, ASC Symposium Series 580, Carbohydrate Modifications in Antisense Research, Sanghui & Cook, eds. Nucleic acids containing one or more carbocyclic sugars are also included within one definition of nucleic acids. Modifications of the ribose-phosphate backbone may be done for a variety of reasons, e.g., to increase the stability and half-life of such molecules in physiological environments or as probes on a biochip. Mixtures of naturally occurring nucleic acids and analogs can be made; alternatively, mixtures of different nucleic acid analogs, and mixtures of naturally occurring nucleic acids and analogs may be made.

A particular nucleic acid sequence also encompasses "splice variants." Similarly, a particular protein encoded by a nucleic acid encompasses any protein encoded by a splice variant of that nucleic acid. "Splice variants," as the name suggests, are products of alternative splicing of a gene. After transcription, an initial nucleic acid transcript may be spliced such that different (alternate) nucleic acid splice products encode different polypeptides. Mechanisms for the production of splice variants vary, but include alternate splicing of exons. Alternate polypeptides derived from the same nucleic acid by read-through transcription are also encompassed by this definition. Any products of a splicing reaction, including recombinant forms of the splice products, are included in this definition. An example of potassium channel splice variants is discussed in Leicher, et al., J. Biol. Chem. 273(52):35095-35101 (1998).

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are near each other, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "probe" or "primer", as used herein, is defined to be one or more nucleic acid fragments whose specific hybridization to a sample can be detected. A probe or primer can be of any length depending on the particular technique it will be used for. For example, PCR primers are generally between 10 and 40 nucleotides in length, while nucleic acid probes for, e.g., a Southern blot, can be more than a hundred nucleotides in length. The probe may be unlabeled or labeled as described below so that its binding to the target or sample can be detected. The probe can be produced from a source of nucleic acids from one or more particular (preselected) portions of a chromosome, e.g., one or more clones, an isolated whole chromosome or chromosome fragment, or a collection of polymerase chain reaction (PCR) amplification products. The length and complexity of the nucleic acid fixed onto the target element is not critical to the invention. One of skill can adjust these factors to provide optimum hybridization and signal production for a given hybridization procedure, and to provide the required resolution among different genes or genomic locations.

The probe may also be isolated nucleic acids immobilized on a solid surface (e.g., nitrocellulose, glass, quartz, fused silica slides), as in an array. The probe may be a member of an array of nucleic acids as described, for instance, in WO 96/17958. Techniques capable of producing high density arrays can also be used for this purpose (see, e.g., Fodor (1991) Science 767-773; Johnston (1998) Curr. Biol. 8: R171-R174; Schummer (1997) Biotechniques 23: 1087-1092; Kern (1997) Biotechniques 23: 120-124; U.S. Patent No. 5,143,854).

A "labeled nucleic acid probe or oligonucleotide" is one that is bound, either covalently, through a linker or a chemical bond, or noncovalently, through ionic, van der Waals, electrostatic, or hydrogen bonds to a label such that the presence of the probe may be detected by detecting the presence of the label bound to the probe. Alternatively, a method using high affinity interactions may achieve the same results where one of a pair of binding partners binds to the other, e.g., biotin, streptavidin.

The terms "identical" or percent sequence "identity," in the context of two or more nucleic acids or polypeptide sequences, refer to two or more sequences or subsequences that are the same or have a specified percentage of amino acid residues or nucleotides that are the same (i.e., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (see, e.g., NCBI web site at ncbi.nlm.nih.gov/BLAST/ or the like). Such sequences are then said to be "substantially identical." This definition also refers to, or may be applied to, the compliment of a test sequence. The definition also includes sequences that have deletions and/or additions, as well as those that have substitutions. Employed algorithms can account for gaps and the like.

For sequence comparisons, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are wellknown in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Current Protocols in Molecular Biology (Ausubel et al., eds. 1995 supplement)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively.

The phrase "selectively (or specifically) hybridizes to" refers to the binding, duplexing, or hybridizing of a molecule only to a particular nucleotide sequence with a higher affinity, e.g., under more stringent conditions, than to other nucleotide sequences (e.g., total cellular or library DNA or RNA).

The phrase "stringent hybridization conditions" refers to conditions under which a nucleic acid will hybridize to its target sequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent hybridization conditions are selected to be about 5-10oC lower than the thermal melting point (Tm) for the specific sequence at a defined ionic strength pH. The Tm is the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent hybridization conditions may also be achieved with the addition of destabilizing agents such as formamide. For selective or specific hybridization, a positive signal is at least two times background, preferably 10 times background hybridization. Exemplary stringent hybridization conditions can be as following: 50% formamide, 5x SSC, and 1% SDS, incubating at 42°C, or, 5x SSC, 1% SDS, incubating at 65°C, with wash in 0.2x SSC, and 0.1% SDS at 65°C. Exemplary "moderately stringent hybridization conditions" include a hybridization in a buffer of 40% formamide, 1 M NaCl, 1% SDS at 37°C, and a wash in IX SSC at 45°C. A positive hybridization is at least twice background. Those of ordinary skill will readily recognize that alternative hybridization and wash conditions can be utilized to provide conditions of similar stringency. Additional guidelines for determining hybridization parameters are provided in numerous reference, e.g., and Current Protocols in Molecular Biology, ed. Ausubel, et al., John Wiley & Sons.

Nucleic acids may be substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions.

An "inhibitory nucleic acid" is a nucleic acid (e.g. DNA, RNA, polymer of nucleotide analogs) that is capable of binding to a target nucleic acid (e.g. an mRNA translatable into PTPRS) and reducing transcription of the target nucleic acid (e.g. mRNA from DNA) or reducing the translation of the target nucleic acid (e.g.mRNA) or altering transcript splicing (e.g. single stranded morpholino oligo). A "morpholino oligo" may be alternatively referred to as a "morphlino nucleic acid" and refers to morpholine-containing nucleic acid nucleic acids commonly known in the art (e.g. phosphoramidate morpholinio oligo or a "PMO"). See Marcos, P., Biochemical and Biophysical Research Communications 358 (2007) 521-527. In some embodiments, the "inhibitory nucleic acid" is a nucleic acid that is capable of binding (e.g. hybridizing) to a target nucleic acid (e.g. an mRNA translatable into an RPTPS) and reducing translation of the target nucleic acid. The target nucleic acid is or includes one or more target nucleic acid sequences to which the inhibitory nucleic acid binds (e.g. hybridizes). Thus, an inhibitory nucleic acid typically is or includes a sequence (also referred to herein as an "antisense nucleic acid sequence") that is capable of hybridizing to at least a portion of a target nucleic acid at a target nucleic acid sequence, An example of an inhibitory nucleic acid is an antisense nucleic acid. Another example of an inhibitory nucleic acid is siRNA or RNAi (including their derivatives or pre-cursors, such as nucleotide analogs). Further examples include shRNA, miRNA, shmiRNA, or certain of their derivatives or pre-cursors. In some embodiments, the inhibitory nucleic acid is single stranded. In other embodiments, the inhibitory nucleic acid is double stranded.

An "antisense nucleic acid" is a nucleic acid (e.g. DNA, RNA or analogs thereof) that is at least partially complementary to at least a portion of a specific target nucleic acid (e.g. a target nucleic acid sequence), such as an mRNA molecule (e.g. a target mRNA molecule) (see, e.g., Weintraub, Scientific American, 262:40 (1990)), for example antisense , siRNA, shRNA, shmiRNA, miRNA (microRNA). Thus, antisense nucleic acids are capable of hybridizing to (e.g. selectively hybridizing to) a target nucleic acid (e.g. target mRNA). In some embodiments, the antisense nucleic acid hybridizes to the target nucleic acid sequence (e.g. mRNA) under stringent hybridization conditions. In some embodiments, the antisense nucleic acid hybridizes to the target nucleic acid (e.g. mRNA) under moderately stringent hybridization conditions. Antisense nucleic acids may comprise naturally occurring nucleotides or modified nucleotides such as, e.g., phosphorothioate, methylphosphonate, and -anomeric sugar-phosphate, backbonemodified nucleotides. An "anti-PTPRS antisense nucleic acid" is an antisense nucleic acid that is at least partially complementary to at least a portion of a target nucleic acid sequence, such as an mRNA molecule, that codes at least a portion of the PTPRS. An antisense nucleic acid may be a morpholino oligo. A morpholino oligo may be a single stranded antisense nucleic acid, as is known in the art. A morpholino oligo may decrease protein expression of a target, reduce translation of the target mRNA, reduce translation initiation of the target mRNA, or modify transcript splicing. The morpholino oligo may be conjugated to a cell permeable moiety (e.g. peptide). Antisense nucleic acids may be single or double stranded nucleic acids.

In the cell, the antisense nucleic acids may hybridize to the target mRNA, forming a double-stranded molecule. The antisense nucleic acids, interfere with the translation of the mRNA, since the cell will not translate a mRNA that is double-stranded. The use of antisense methods to inhibit the in vitro translation of genes is well known in the art (Marcus-Sakura, Anal. Biochem., 172:289, (1988)). Antisense molecules which bind directly to the DNA may be used.

Inhibitory nucleic acids can be delivered to the subject using any appropriate means known in the art, including by injection, inhalation, or oral ingestion. Another suitable delivery system is a colloidal dispersion system such as, for example, macromolecule complexes, nanocapsules, microspheres, beads, and lipid-based systems including oil-in-water emulsions, micelles, mixed micelles, and liposomes. An example of a colloidal system of this invention is a liposome. Liposomes are artificial membrane vesicles which are useful as delivery vehicles in vitro and in vivo. Nucleic acids, including RNA and DNA within liposomes and be delivered to cells in a biologically active form (Fraley, et al., Trends Biochem. Sci., 6:77, 1981). Liposomes can be targeted to specific cell types or tissues using any means known in the art. Inhibitory nucleic acids (e.g. antisense nucleic acids, morpholino oligos) may be delivered to a cell using cell permeable delivery systems (e.g. cell permeable peptides). Inhibitory nucleic acids may be delivered to specific cells or tissues using viral vectors or viruses.

An "siRNA" refers to a nucleic acid that forms a double stranded RNA, which double stranded RNA has the ability to reduce or inhibit expression of a gene or target gene when the siRNA is present (e.g. expressed) in the same cell as the gene or target gene. The siRNA is typically about 5 to about 100 nucleotides in length, more typically about 10 to about 50 nucleotides in length, more typically about 15 to about 30 nucleotides in length, most typically about 20-30 base nucleotides, or about 20-25 or about 24-29 nucleotides in length, e.g., 20, 21, 22, 23, 24, 25, 26, 27, 28, 29, or 30 nucleotides in length. siRNA molecules and methods of generating them are described in, e.g., Bass, 2001, Nature, 411, 428-429; Elbashir et al., 2001, Nature, 411, 494-498; WO 00/44895; WO 01/36646; WO 99/32619; WO 00/01846; WO 01/29058; WO 99/07409; and WO 00/44914. A DNA molecule that transcribes dsRNA or siRNA (for instance, as a hairpin duplex) also provides RNAi. DNA molecules for transcribing dsRNA are disclosed in U.S. Patent No. 6,573,099, and in U.S. Patent Application Publication Nos. 2002/0160393 and 2003/0027783, and Tuschl and Borkhardt, Molecular Interventions, 2:158 (2002).

The siRNA can be administered directly or siRNA expression vectors can be used to induce RNAi that have different design criteria. A vector can have inserted two inverted repeats separated by a short spacer sequence and ending with a string of T's which serve to terminate transcription.

Construction of suitable vectors containing the nucleic acid sequences employs standard ligation and restriction techniques, which are well understood in the art (see Maniatis et al., in Molecular Cloning: A Laboratory Manual, Cold Spring Harbor Laboratory, New York (1982)). Isolated plasmids, DNA sequences, or synthesized oligonucleotides are cleaved, tailored, and re-ligated in the form desired.

"Biological sample" or "sample" refer to materials obtained from or derived from a subject or patient. A biological sample includes sections of tissues such as biopsy and autopsy samples, and frozen sections taken for histological purposes. Such samples include bodily fluids such as blood and blood fractions or products (e.g., serum, plasma, platelets, red blood cells, and the like), sputum, tissue, cultured cells (e.g., primary cultures, explants, and transformed cells) stool, urine, synovial fluid, joint tissue, synovial tissue, synoviocytes, fibroblast-like synoviocytes, macrophage-like synoviocytes, immune cells, hematopoietic cells, fibroblasts, macrophages, T cells, etc. A biological sample is typically obtained from a eukaryotic organism, such as a mammal such as a primate e.g., chimpanzee or human; cow; dog; cat; a rodent, e.g., guinea pig, rat, mouse; rabbit; or a bird; reptile; or fish.

A "biopsy" refers to the process of removing a tissue sample for diagnostic or prognostic evaluation, and to the tissue specimen itself. Any biopsy technique known in the art can be applied to the diagnostic and prognostic methods described herein. The biopsy technique applied will depend on the tissue type to be evaluated (i.e., prostate, lymph node, liver, bone marrow, blood cell, joint tissue, synovial tissue, synoviocytes, fibroblast-like synoviocytes, macrophage-like synoviocytes, immune cells, hematopoietic cells, fibroblasts, macrophages, T cells, etc.), among other factors. Representative biopsy techniques include excisional biopsy, incisional biopsy, needle biopsy, surgical biopsy, and bone marrow biopsy. Biopsy techniques are discussed, for example, in Harrison's Principles of Internal Medicine, Kasper, et al., eds., 16th ed., 2005, Chapter 70, and throughout Part V.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an α carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

A "label" or a "detectable moiety" is a composition detectable by spectroscopic, photochemical, biochemical, immunochemical, chemical, or other physical means. For example, useful labels include 32P, fluorescent dyes, electron-dense reagents, enzymes (e.g., as commonly used in an ELISA), biotin, digoxigenin, or haptens and proteins or other entities which can be made detectable, e.g., by incorporating a radiolabel into a peptide or antibody specifically reactive with a target peptide. Any method known in the art for conjugating an antibody to the label may be employed, e.g., using methods described in Hermanson, Bioconjugate Techniques 1996, Academic Press, Inc., San Diego.

The term "recombinant" when used with reference, e.g., to a cell, or nucleic acid, protein, or vector, indicates that the cell, nucleic acid, protein or vector has been modified by or is the result oflaboratory methods. Thus, for example, recombinant proteins include proteins produced by laboratory methods. Recombinant proteins can include amino acid residues not found within the native (non-recombinant) form of the protein or can be include amino acid residues that have been modified, e.g., labeled.

The term "heterologous" when used with reference to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, e.g., a promoter from one source and a coding region from another source. Similarly, a heterologous protein indicates that the protein comprises two or more subsequences that are not found in the same relationship to each other in nature (e.g., a fusion protein).

"Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody will be most critical in specificity and affinity of binding. In some embodiments, antibodies or fragments of antibodies may be derived from different organisms, including humans, mice, rats, hamsters, camels, etc. Antibodies of the invention may include antibodies that have been modified or mutated at one or more amino acid positions to improve or modulate a desired function of the antibody (e.g. glycosylation, expression, antigen recognition, effector functions, antigen binding, specificity, etc.).

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains respectively.

Antibodies exist, e.g., as intact immunoglobulins or as a number of well-characterized fragments produced by digestion with various peptidases. Thus, for example, pepsin digests an antibody below the disulfide linkages in the hinge region to produce F(ab)'2, a dimer of Fab which itself is a light chain joined to VH-CH1 by a disulfide bond. The F(ab)'2 may be reduced under mild conditions to break the disulfide linkage in the hinge region, thereby converting the F(ab)'2 dimer into an Fab' monomer. The Fab' monomer is essentially Fab with part of the hinge region (see Fundamental Immunology (Paul ed., 3d ed. 1993). While various antibody fragments are defined in terms of the digestion of an intact antibody, one of skill will appreciate that such fragments may be synthesized de novo either chemically or by using recombinant DNA methodology. Thus, the term antibody, as used herein, also includes antibody fragments either produced by the modification of whole antibodies, or those synthesized de novo using recombinant DNA methodologies (e.g., single chain Fv) or those identified using phage display libraries (see, e.g., McCafferty et al., Nature 348:552-554 (1990)).

For preparation of suitable antibodies for use according to the invention, e.g., recombinant, monoclonal, or polyclonal antibodies, many techniques known in the art can be used (see, e.g., Kohler & Milstein, Nature 256:495-497 (1975); Kozbor et al., Immunology Today 4: 72 (1983); Cole et al., pp. 77-96 in Monoclonal Antibodies and Cancer Therapy, Alan R. Liss, Inc. (1985); Coligan, Current Protocols in Immunology (1991); Harlow & Lane, Antibodies, A Laboratory Manual (1988); and Goding, Monoclonal Antibodies: Principles and Practice (2d ed. 1986)). The genes encoding the heavy and light chains of an antibody of interest can be cloned from a cell, e.g., the genes encoding a monoclonal antibody can be cloned from a hybridoma and used to produce a recombinant monoclonal antibody. Gene libraries encoding heavy and light chains of monoclonal antibodies can also be made from hybridoma or plasma cells. Random combinations of the heavy and light chain gene products generate a large pool of antibodies with different antigenic specificity (see, e.g., Kuby, Immunology (3rd ed. 1997)). Techniques for the production of single chain antibodies or recombinant antibodies (U.S. Patent 4,946,778, U.S. Patent No. 4,816,567) can be adapted to produce antibodies to polypeptides of this invention. Also, transgenic mice, or other organisms such as other mammals, may be used to express humanized or human antibodies (see, e.g., U.S. Patent Nos. 5,545,807; 5,545,806; 5,569,825; 5,625,126; 5,633,425; 5,661,016, Marks et al., Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); and Lonberg & Huszar, Intern. Rev. Immunol. 13:65-93 (1995)). Alternatively, phage display technology can be used to identify antibodies and heteromeric Fab fragments that specifically bind to selected antigens (see, e.g., McCafferty et al., Nature 348:552-554 (1990); Marks et al., Biotechnology 10:779-783 (1992)). Antibodies can also be made bispecific, i.e., able to recognize two different antigens (see, e.g., WO 93/08829, Traunecker et al., EMBO J. 10:3655-3659 (1991); and Suresh et al., Methods in Enzymology 121:210 (1986)). Antibodies can also be heteroconjugates, e.g., two covalently joined antibodies, or immunotoxins (see, e.g., U.S. Patent No. 4,676,980 , WO 91/00360; WO 92/200373; and EP 03089).

Methods for humanizing or primatizing non-human antibodies are well known in the art (e.g., U.S. Patent Nos. 4,816,567; 5,530,101; 5,859,205; 5,585,089; 5,693,761; 5,693,762; 5,777,085; 6,180,370; 6,210,671; and 6,329,511; WO 87/02671; EP Patent Application 0173494; Jones et al. (1986) Nature 321:522; and Verhoyen et al. (1988) Science 239:1534). Humanized antibodies are further described in, e.g., Winter and Milstein (1991) Nature 349:293. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers (see, e.g., Morrison et al., PNAS USA, 81:6851-6855 (1984), Jones et al., Nature 321:522-525 (1986); Riechmann et al., Nature 332:323-327 (1988); Morrison and Oi, Adv. Immunol., 44:65-92 (1988), Verhoeyen et al., Science 239:1534-1536 (1988) and Presta, Curr. Op. Struct. Biol. 2:593-596 (1992), Padlan, Molec. Immun., 28:489-498 (1991); Padlan, Molec. Immun., 31(3):169-217 (1994)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies. For example, polynucleotides comprising a first sequence coding for humanized immunoglobulin framework regions and a second sequence set coding for the desired immunoglobulin complementarity determining regions can be produced synthetically or by combining appropriate cDNA and genomic DNA segments. Human constant region DNA sequences can be isolated in accordance with well known procedures from a variety of human cells.

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. The preferred antibodies of, and for use according to the invention include humanized and/or chimeric monoclonal antibodies.

Techniques for conjugating therapeutic agents to antibodies are well known (see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery"in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review" in Monoclonal Antibodies '84: Biological And Clinical Applications, Pinchera et al. (eds.), pp. 475-506 (1985); and Thorpe et al., "The Preparation And Cytotoxic Properties Of Antibody-Toxin Conjugates", Immunol. Rev., 62:119-58 (1982)).

The phrase "specifically (or selectively) binds" to an antibody or "specifically (or selectively) immunoreactive with," when referring to a protein or peptide, refers to a binding reaction that is determinative of the presence of the protein, often in a heterogeneous population of proteins and other biologics. Thus, under designated immunoassay conditions, the specified antibodies bind to a particular protein at least two times the background and more typically more than 10 to 100 times background. Specific binding to an antibody under such conditions requires an antibody that is selected for its specificity for a particular protein. For example, polyclonal antibodies can be selected to obtain only those polyclonal antibodies that are specifically immunoreactive with the selected antigen and not with other proteins. This selection may be achieved by subtracting out antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Using Antibodies, A Laboratory Manual (1998) for a description of immunoassay formats and conditions that can be used to determine specific immunoreactivity).

As used herein, the term "pharmaceutically acceptable" is used synonymously with "physiologically acceptable" and "pharmacologically acceptable". A pharmaceutical composition will generally comprise agents for buffering and preservation in storage, and can include buffers and carriers for appropriate delivery, depending on the route of administration.

Certain compounds used in the present invention can exist in unsolvated forms as well as solvated forms, including hydrated forms. In general, the solvated forms are equivalent to unsolvated forms and are intended to be encompassed within the scope of the present invention. Certain compounds used in the present invention may exist in multiple crystalline or amorphous forms. In general, all physical forms are equivalent for the uses contemplated by the present invention and are intended to be within the scope of the present invention.

"PTPR" or "RPTP" or "rPTP" (all terms are equal) refer to receptor protein tyrosine phosphatases, which are found in nature as protein tyrosine phosphatases.

"PTPRS" refers to protein tyrosine phosphatase receptor type S (or sigma), which is a member of the protein tyrosine phosphatase (PTP) family. The amino acid sequence of PTPRS can be found, for example, at UniProtKB/Swiss-Prot Accession No. Q13332 and B0V2N1, and also SEQ ID NO:4. The nucleic acid sequence of PTPRS can be found, for example, at GenBank Accession No. NC_000019.9 and. PTPRS includes an intracellular domain, e.g., amino acid residues 1304-1948 of SEQ ID NO:8 or amino acid residues 1279-1907 of SEQ ID NO:4, a transmembrane domain, e.g., amino acid residues 1283-1303 of SEQ ID NO:8 or amino acid residues 1258-1278 of SEQ ID NO:4, and an extracellular domain, e.g., SEQ ID NO:9 or SEQ ID NO:10. The term transmembrane domain refers to the portion of a protein or polypeptide that is embedded in and, optionally, spans a membrane. The term intracellular domain refers to the portion of a protein or polypeptide that extends into the cytoplasm of a cell. The term extracellular domain refers to the portion of a protein or polypeptide that extends into the extracellular environment. The extracellular domain of PTPRS includes immunoglobulin-like domain 1 (Ig1), immunoglobulin-like domain 2 (Ig2) and immunoglobulin-like domain 2 (Ig3). The amino acid sequence of Ig1 includes amino acid residues 30 to 127 of SEQ ID NO:4 or amino acid residues 30-127 of SEQ ID NO:8, or the amino acid sequence EEPRFIKEPKDQIGVSGGVASFVCQATGDPKPRVTWNKKGKKVNSQRFETIEFDESAGA VLRIQPLRTPRDENVYECVAQNSVGEITVHAKLTVLRE (SEQ ID NO:1) or the amino acid sequence of SEQ ID NO:5. The amino acid sequence of Ig2 includes amino acid residues 128 to 231 of SEQ ID NO:4, or amino acid residues 128-244 of SEQ ID NO:8, or the amino acid sequence DQLPSGFPNIDMGPQLKVVERTRTATMLCAASGNPDPEITWFKDFLPVDPSASNGRIKQL RSETFESTPIRGALQIESSEETDQGKYECVATNSAGVRYSSPANLYVRVRRVA (SEQ ID NO:2) or the amino acid sequence of SEQ ID NO:6. The amino acid sequence of Ig3 includes amino acid residues 232 to 321 of SEQ ID NO:4, or amino acid residues 245-334 of SEQ ID NO:8 or the amino acid sequence PRFSILPMSHEIMPGGNVNITCVAVGSPMPYVKWMQGAEDLTPEDDMPVGRNVLELTD VKDSANYTCVAMSSLGVIEAVAQITVKSLPKA (SEQ ID NO:3) or the amino acid sequence of SEQ ID NO:7.

A "protein level of an RPTP" refers to an amount (relative or absolute) of RPTP in its protein form (as distinguished from its precursor RNA form). A protein of an RPTP may include a full-length protein (e.g. the protein translated from the complete coding region of the gene, which may also include post-translational modifications), functional fragments of the full length protein (e.g. sub-domains of the full length protein that possess an activity or function in an assay), or protein fragments of the RPTP, which may be any peptide or oligopeptide of the full length protein.

An "RNA level of an RPTP" refers to an amount (relative or absolute) of RNA present that may be translated to form an RPTP. The RNA of an RPTP may be a full-length RNA sufficient to form a full-length RPTP. The RNA of an RPTP may also be a fragment of the full length RNA thereby forming a fragment of the full length RPTP. The fragment of the full length RNA may form a functional fragment of the RPTP. The RNA of an RPTP may include all splice variants of an RPTP gene.

An "autoimmune therapeutic agent" is a molecule (e.g. antibody, nucleic acid, inhibitory nucleic acid, synthetic chemical, small chemical molecule) that treats or prevents an autoimmune disease when administered to a subject in a therapeutically effective dose or amount. An autoimmune therapeutic agent may be an RPTP binding agent.

An "RPTP binding agent" is a molecule that binds (e.g. preferentially binds) to RPTP, RNA that is translatable to RPTP, or DNA that is transcribable to an RNA that is translatable to an RPTP. Where the molecule preferentially binds, the binding is preferential as compared to other macromolecular biomolecules present in an organism or cell. A compound preferentially binds to as compared to other macromolecular biomolecules present in an organism or cell, for example, when the preferential binding is 1.1-fold, 1.2-fold, 1.3-fold, 1.4-fold, 1.5-fold, 1.6-fold, 1.7-fold, 1.8-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, 200-fold, 300-fold, 400-fold, 500-fold, 600-fold, 700-fold, 800-fold, 900-fold, 1000-fold, 2000-fold, 3000-fold, 4000-fold, 5000-fold, 6000-fold, 7000-fold, 8000-fold, 9000-fold, 10000-fold, 100,000-fold, 1,000,000-fold greater.

An agent may "target" an RPTP, a nucleic acid (e.g. RNA or DNA) encoding an RPTP, or a protein of an RPTP, by binding (e.g. preferentially binding) to the RPTP, nucleic acid (e.g. RNA or DNA) encoding an RPTP, or protein of an RPTP. Optionally, the RPTP is PTPRS. An agent preferentially binds to a molecule, for example, when the binding to the targeted molecule is greater than the binding to other molecules of a similar form. The preferential binding may be 1.1-fold, 1.2-fold, 1.3-fold, 1.4-fold, 1.5-fold, 1.6-fold, 1.7-fold, 1.8-fold, 1.9-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, 200-fold, 300-fold, 400-fold, 500-fold, 600-fold, 700-fold, 800-fold, 900-fold, 1000-fold, 2000-fold, 3000-fold, 4000-fold, 5000-fold, 6000-fold, 7000-fold, 8000-fold, 9000-fold, 10000 fold, 100,000-fold, 1,000,000-fold greater. In some instances, an agent targets an RPTP, a nucleic acid (e.g. RNA or DNA) of an RPTP, or a protein of an RPTP when a binding assay or experiment (e.g. gel electrophoresis, chromatography, immunoassay, radioactive or non-radioactive labeling, immunoprecipitation, activity assay, etc.) reveals only an interaction or primarily an interaction with a single RPTPS, a nucleic acid (e.g. RNA or DNA) of a single RPTP, or a protein of a single RPTP. An agent may also "target" an RPTP, a nucleic acid (e.g. RNA or DNA) of an RPTP, or a protein of an RPTPS by binding to the RPTP, nucleic acid (e.g. RNA or DNA) of an RPTP, or protein of an RPTP, by decreasing or increasing the amount of RPTP in a cell or organism relative to the absence of the agent, or decreasing the interaction between the RPTP with a physiological or natural ligand. A person having ordinary skill in the art, using the guidance provided herein, may easily determine whether an agent decreases or increases the amount of an RPTP in a cell or organism.

As used herein, "treating" or "treatment of' a condition, disease or disorder or symptoms associated with a condition, disease or disorder refers to an approach for obtaining beneficial or desired results, including clinical results. Beneficial or desired clinical results can include, but are not limited to, alleviation or amelioration of one or more symptoms or conditions, diminishment of extent of condition, disorder or disease, stabilization of the state of condition, disorder or disease, prevention of development of condition, disorder or disease, prevention of spread of condition, disorder or disease, delay or slowing of condition, disorder or disease progression, delay or slowing of condition, disorder or disease onset, amelioration or palliation of the condition, disorder or disease state, and remission, whether partial or total. "Treating" can also mean prolonging survival of a subject beyond that expected in the absence of treatment. "Treating" can also mean inhibiting the progression of the condition, disorder or disease, slowing the progression of the condition, disorder or disease temporarily, although in some instances, it involves halting the progression of the condition, disorder or disease permanently. As used herein the terms treatment, treat, or treating refers to a method of reducing the effects of one or more symptoms of a disease or condition characterized by expression of the protease or symptom of the disease or condition characterized by expression of the protease. Thus in the disclosed method, treatment can refer to a 10%, 20%, 30%, 40%, 50%, 60%, 70%, 80%, 90%, or 100% reduction in the severity of an established disease, condition, or symptom of the disease or condition. For example, a method for treating a disease is considered to be a treatment if there is a 10% reduction in one or more symptoms of the disease in a subject as compared to a control. Thus the reduction can be a 10%, 20%, 30%, 40%, 50%, 60%, 70%, 80%, 90%, 100%, or any percent reduction in between 10% and 100% as compared to native or control levels. It is understood that treatment does not necessarily refer to a cure or complete ablation of the disease, condition, or symptoms of the disease or condition. Further, as used herein, references to decreasing, reducing, or inhibiting include a change of 10%, 20%, 30%, 40%, 50%, 60%, 70%, 80%, 90% or greater as compared to a control level and such terms can include but do not necessarily include complete elimination.

Described herein are PTPRS de-clustering agents. As used herein, the terms "PTPRS de-clustering agent" and the like refer to an agent (e.g., small molecules, proteins including antibodies, and the like) capable of causing a reduction in the level of dimerization, oligomerization or clustering of PTPRS proteins. Without wishing to be bound by any theory, it is believed that clustering of PTPRS can give rise to an inactive dimeric form. Accordingly, the action of a PTPRS de-clustering agent results in monomeric PTPRS which regains activity relative to the clustered (e.g., dimerized or oligomerized) form of PTPRS. Optionally, the PTPRS de-clustering agent is a non-enzymatic recombinant protein comprising an amino acid sequence of an extracellular domain of PTPRS or a subsequence, portion, homologue, variant or derivative thereof, as described herein. Optionally, the non-enzymatic recombinant protein is the extracellular domain of PTPRS. Without being limited to any particular theory, the extracellular domain of PTPRS or portions thereof displaces PTPRS from HS. This can activate PTPRS and lead to dephosphorylation of beta-catenin and other substrates and inhibition of downstream FLS invasiveness and pro-inflammatory actions. This is supported by the examples and data provided herein. Optionally, the PTPRS de-clustering agent is an anti-PRPRS antibody or fragment thereof. Optionally, the PTPRS de-clustering agent is an anti-PTPRS aptamer. Optionally, the PTPRS de-clustering agent binds heparan sulfate. Optionally, the PTPRS de-clustering agent is an anti-heparan sulfate antibody or fragment thereof. Optionally, the PTPRS de-clustering agent is an anti-heparan sulfate aptamer. Optionally, the PTPRS de-clustering agent is not chondroitin sulfate. The PTPRS is, optionally, not a chondroitin sulfate mimetic or an agent that has the same or similar mechanism of action as chondroitin sulfate. In other embodiments, the PTPRS de-clustering agent is a chondroitin sulfate mimetic.

Provided herein are non-enzymatic recombinant proteins comprising an amino acid sequence of an extracellular domain of PTPRS, or a subsequence, portion, homologue, variant or derivative thereof. As used herein, the term "non-enzymatic recombinant protein" refers to a recombinant protein that does not have enzymatic activity (e.g., the protein does not function as a biological catalyst). Thus, in some embodiments, the non-enzymatic recombinant proteins comprising an amino acid sequence of an extracellular domain of PTPRS provided herein include only extracellular domain portions of the PTPRS and not the enzymatic portions of the PTPRS. In embodiments, the non-enzymatic recombinant proteins comprising an amino acid sequence of an extracellular domain of PTPRS provided herein include only extracellular domain portions of the PTPRS and not the enzymatic portions of the PTPRS or the transmembrane portions of the PTPRS. In some embodiments, the non-enzymatic recombinant proteins comprising an amino acid sequence of an extracellular domain of PTPRS include two or more extracellular domain of PTPRS linked together (e.g. linked by an amino acid linker such as an amino acid linker of at least 2, at least 3, at least 5, at least 10, about 2 to 50 or 100 amino acids, about 3 to 50 or 100 amino acids, or about 2, 3,4 5, 6, 7, 8, 9, 10, 20, 30, 40, 50, 60, 70, 80, 90, or 100 amino acides wherein the amino acid sequence is designed to not interfere with extracellular domain of PTPRS ligand binding). The term "extracellular domain of PTPRS" can include subsequences, portions, homologues, variants or derivatives of the extracellular domain of PTPRS. Thus, the non-enzymatic recombinant protein can comprise a portion of the extracellular domain of PTPRS, e.g., the protein comprises one or more immunoglobulin-like domains of PTPRS, e.g., SEQ ID NO:1, SEQ ID NO:2, SEQ ID NO:3, SEQ ID NO:5, SEQ ID NO:6 and/or SEQ ID NO:7, or subsequences, portions, homologues, variants or derivatives thereof. The extracellular domain of PTPRS is typically capable of binding (e.g specifically binding) to a PTPRS ligand such as heparan sulfate. The extracellular domain of PTPRS comprises both PTPRS immunoglobulin-like domain 1 (Ig1) and immunoglobulin-like domain 2 (Ig2). The extracellular domain may also comprise immunoglobulin-like domain 3 (Ig3), or a subsequence, portion, homologue, variant or derivative thereof.

Optionally, the protein comprises Ig1 amino acid residues 39 to 124 of SEQ ID NO:4, or a subsequence, portion, homologue, variant or derivative thereof. Optionally, the protein comprises an amino acid sequence set forth as:
EEPRFIKEPKDQIGVSGGVASFVCQATGDPKPRVTWNKKGKKVNSQRFETIEFDESAGA VLRIQPLRTPRDENVYECVAQNSVGEITVHAKLTVLRE(SEQ ID NO:1) or set forth as SEQ ID NO:5, or a subsequence, portion, homologue, variant or derivative thereof.

Optionally, the protein comprises Ig2 amino acid residues 152 to 233 of SEQ ID NO:4, or a subsequence, portion, homologue, variant or derivative thereof. Optionally, the protein comprises an amino acid sequence set forth as:
DQLPSGFPNIDMGPQLKVVERTRTATMLCAASGNPDPEITWFKDFLPVDPSASNGRIKQL RSETFESTPIRGALQIESSEETDQGKYECVATNSAGVRYSSPANLYVRVRRVA (SEQ ID NO:2) or set forth as SEQ ID NO:6, or a subsequence, portion, homologue, variant or derivative thereof.

Optionally, the protein comprises Ig3 amino acid residues 259-327 of SEQ ID NO:4, or a subsequence, portion, homologue, variant or derivative thereof. Optionally, the protein comprises an amino acid sequence set forth as:
PRFSILPMSHEIMPGGNVNITCVAVGSPMPYVKWMQGAEDLTPEDDMPVGRNVLELTD VKDSANYTCVAMSSLGVIEAVAQITVKSLPKA (SEQ ID NO:3), or set forth as SEQ ID NO:7, or a subsequence, portion, homologue, variant or derivative thereof.

In some embodiments, the non-enzymatic recombinant protein comprising an amino acid sequence of an extracellular domain of PTPRS or portion thereof lacks a transmembrane domain and/or lacks an intracellular domain. In some embodiments, the non-enzymatic recombinant protein comprising an amino acid sequence of an extracellular domain of PTPRS or portion thereof lacks a transmembrane domain. In some embodiments, the non-enzymatic recombinant protein comprising an amino acid sequence of an extracellular domain of PTPRS or portion thereof lacks an intracellular domain.

Optionally, the provided PTPRS de-clustering agent binds (e.g. specifically binds) heparan sulfate. Optionally, the PTPRS de-clustering agent prevents oligomerization or clustering of PTPRS proteins; for example, the PTPRS de-clustering agent prevents dimerization of PTPRS proteins. Optionally, the PTPRS de-clustering agent modulates PTPRS activity; for example, the PTPRS de-clustering agent increases the phosphatase activity of PTPRS.

Provided herein are compositions including the agents provided herein. Provided herein are pharmaceutical compositions including a PTPRS de-clustering agent and a pharmaceutically acceptable excipient. Provided compositions can include a single agent or more than one agent. The provided compositions are, optionally, suitable for formulation and administration *in vitro* or *in vivo.* Optionally, the compositions comprise one or more of the provided agents and a pharmaceutically acceptable carrier. Suitable carriers and their formulations are described in Remington: The Science and Practice of Pharmacy, 21st Edition, David B. Troy, ed., Lippicott Williams & Wilkins (2005). By pharmaceutically acceptable carrier is meant a material that is not biologically or otherwise undesirable, i.e., the material is administered to a subject without causing undesirable biological effects or interacting in a deleterious manner with the other components of the pharmaceutical composition in which it is contained. If administered to a subject, the carrier is optionally selected to minimize degradation of the active ingredient and to minimize adverse side effects in the subject.

The term "pharmaceutically acceptable salts" or "pharmaceutically acceptable carrier" is meant to include salts of the active compounds which are prepared with relatively nontoxic acids or bases, depending on the particular substituents found on the compounds described herein. When compounds of the present application contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present application contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, e.g., Berge et al., Journal of Pharmaceutical Science 66:1-19 (1977)). Other pharmaceutically acceptable carriers known to those of skill in the art are suitable for compositions described herein.

The compositions for administration will commonly comprise an agent as described herein dissolved in a pharmaceutically acceptable carrier, preferably an aqueous carrier. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of active agent in these formulations can vary widely, and will be selected primarily based on fluid volumes, viscosities, body weight and the like in accordance with the particular mode of administration selected and the subject's needs.

Solutions of the active compounds as free base or pharmacologically acceptable salt can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations can contain a preservative to prevent the growth of microorganisms.

Pharmaceutical compositions can be delivered via intranasal or inhalable solutions or sprays, aerosols or inhalants. Nasal solutions can be aqueous solutions designed to be administered to the nasal passages in drops or sprays. Nasal solutions can be prepared so that they are similar in many respects to nasal secretions. Thus, the aqueous nasal solutions usually are isotonic and slightly buffered to maintain a pH of 5.5 to 6.5. In addition, antimicrobial preservatives, similar to those used in ophthalmic preparations and appropriate drug stabilizers, if required, may be included in the formulation. Various commercial nasal preparations are known and can include, for example, antibiotics and antihistamines.

Oral formulations can include excipients as, for example, pharmaceutical grades of mannitol, lactose, starch, magnesium stearate, sodium saccharine, cellulose, magnesium carbonate and the like. These compositions take the form of solutions, suspensions, tablets, pills, capsules, sustained release formulations or powders. In some embodiments, oral pharmaceutical compositions will comprise an inert diluent or assimilable edible carrier, or they may be enclosed in hard or soft shell gelatin capsule, or they may be compressed into tablets, or they may be incorporated directly with the food of the diet. For oral therapeutic administration, the active compounds may be incorporated with excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 75% of the weight of the unit, or preferably between 25-60%. The amount of active compounds in such compositions is such that a suitable dosage can be obtained

For parenteral administration in an aqueous solution, for example, the solution should be suitably buffered and the liquid diluent first rendered isotonic with sufficient saline or glucose. Aqueous solutions, in particular, sterile aqueous media, are especially suitable for intravenous, intramuscular, subcutaneous and intraperitoneal administration. For example, one dosage could be dissolved in 1 ml of isotonic NaCl solution and either added to 1000 ml of hypodermoclysis fluid or injected at the proposed site of infusion

Sterile injectable solutions can be prepared by incorporating the active compounds or constructs in the required amount in the appropriate solvent followed by filtered sterilization. Generally, dispersions are prepared by incorporating the various sterilized active ingredients into a sterile vehicle which contains the basic dispersion medium. Vacuum-drying and freeze-drying techniques, which yield a powder of the active ingredient plus any additional desired ingredients, can be used to prepare sterile powders for reconstitution of sterile injectable solutions. The preparation of more, or highly, concentrated solutions for direct injection is also contemplated. DMSO can be used as solvent for extremely rapid penetration, delivering high concentrations of the active agents to a small area.

The formulations of compounds can be presented in unit-dose or multi-dose sealed containers, such as ampules and vials. Thus, the composition can be in unit dosage form. In such form the preparation is subdivided into unit doses containing appropriate quantities of the active component. Thus, the compositions can be administered in a variety of unit dosage forms depending upon the method of administration. For example, unit dosage forms suitable for oral administration include, but are not limited to, powder, tablets, pills, capsules and lozenges.

Compositions can be formulated to provide quick, sustained or delayed release after administration by employing procedures known in the art. Certain carriers may be more preferable depending upon, for instance, the route of administration and concentration of composition being administered. Suitable formulations for use in the provided compositions can be found in Remington: The Science and Practice of Pharmacy, 21st Edition, David B. Troy, ed., Lippicott Williams & Wilkins (2005).

Provided herein are kits comprising one or more of the provided compositions and instructions for use. Optionally, the kit comprises one or more doses of an effective amount of a composition comprising a PTPRS de-clustering agent. Optionally, the kit comprises a non-enzymatic recombinant protein comprising an amino acid sequence of an extracellular domain of PTPRS or a subsequence, portion, homologue, variant or derivative thereof. Optionally, the kit comprises one or more portions of the extracellular domain of PTPRS. Optionally, the composition or protein is present in a container (e.g., vial or packet). Optionally, the kit comprises one or more additional agents for treating or preventing one or more symptom of an inflammatory and/or autoimmune disease. Optionally, the kit comprises a means of administering the composition, such as, for example, a syringe, needle, tubing, catheter, patch, and the like. The kit may also comprise formulations and/or materials requiring sterilization and/or dilution prior to use.

The compositions and agents as described herein are useful for both prophylactic and therapeutic treatment. For prophylactic use, a therapeutically effective amount of the agents described herein are administered to a subject prior to or during early onset (e.g., upon initial signs and symptoms of an autoimmune disease). Therapeutic treatment involves administering to a subject a therapeutically effective amount of the agents described herein after diagnosis or development of disease.

The provided proteins, agents and compositions are for use in the treatment of a subject who has or is at risk of developing an autoimmune disease, including for example arthritis such as rheumatoid arthritis or osteoarthritis,. Optionally, the proteins, agents, and compositions are for use in the treatment of a subject who has or is at risk of developing an extracellular matrix disease and/or a fibroblast-mediated disease. As used herein, the term "extracellular matrix disease" refers to a condition, disorder or disease, associated with the extracellular matrix (ECM) or one or more components of the extracellular matrix. The extracellular matrix provides structural support to cells in addition to being involved in other biological functions including, but not limited to, intracellular communication. Components of the extracellular matrix include, but are not limited to, proteoglycans (e.g., heparan sulfate, chondroitin sulfate, and keratin sulfate), non-proteoglycan polysaccharaides (e.g., hyaluronic acid), fibers, collagen, elastin, fibronectin and laminin. The extracellular matrix also serves as a depot for signaling molecules such as growth factors and cytokines. Extracellular matrix diseases include diseases associated with the dysregulation of one or more functions of the ECM (e.g., dysregulated intracellular communication and/or movement) or dysreguation of one or more components of the ECM (e.g., increased or decreased activity and/or production of one or more components of the ECM). Extracellular matrix diseases also include diseases associated with altered degradation and remodeling of the ECM and diseases associated with altered (e.g., increased or decreased) accumulation of agents, e.g., immunocomplexes and other immune products, in the ECM. Extracellular matrix diseases include, but are not limited to, atherosclerosis, cancer, amyloid diseases, glomerular diseases, mesangial diseases, inflammatory conditions, and developmental disorders. As used herein, the term "fibroblast-mediated disease" refers to a condition, disorder, or disease, associated with fibroblast cell activity or movement. Fibroblasts are a type of cell involved in the synthesis of the ECM and collagen and are the major cell type of connective tissue. Types of fibroblasts include, but are not limited to, synovial fibroblasts, dermal fibroblasts, and interstitial fibroblasts. The main function of fibroblasts is to maintain the integrity of connective tissue by continuously secreting components of the ECM. Fibroblast-mediated diseases include diseases associated with the altered activity and/or movement of fibroblasts. Thus, for example, a fibroblast-mediated disease includes diseases associated with altered fibroblast migration or altered fibroblast activity. Fibroblast activities include, but are not limited to, collagen production, glycosaminoglycan production, reticular and elastic fiber production, cytokine production, and glycoprotein production. Thus, fibroblast-mediated diseases include diseases associated with altered production by fibroblasts of one or more of collagen, glycosaminoglycans, reticular and elastic fibers, cytokines, and glycoproteins.

Provided herein are methods of modulating PTPRS activity in a subject, the method comprising administering to the subject an effective amount of a PTPRS de-clustering agent, wherein administration modulates PTPRS activity in the subject. Also provided are methods of treating, preventing, and/or ameliorating an autoimmune disease or disorder in a subject in need thereof. Specifically, provided is a method of treating an autoimmune disease in a subject, the method comprising administering to the subject a therapeutically effective amount of a PTPRS de-clustering agent, wherein administration treats the autoimmune disease in the subject. Also provided is a method of treating an autoimmune disease in a subject, the method comprising administering to the subject a therapeutically effective amount of a compound that increases PTPRS phosphatase activity, wherein administration treats the autoimmune disease in the subject. Autoimmune diseases or disorders include, but are not limited to, inflammatory autoimmune diseases. Optionally, the autoimmune disease is arthritis, rheumatoid arthritis, psoriatic arthritis, juvenile idiopathic arthritis, scleroderma, systemic scleroderma, multiple sclerosis, systemic lupus erythematosus (SLE), myasthenia gravis, juvenile onset diabetes, diabetes mellitus type 1, Guillain-Barre syndrome, Hashimoto's encephalitis, Hashimoto's thyroiditis, ankylosing spondylitis, psoriasis, Sjogren's syndrome,vasculitis, glomerulonephritis, auto-immune thyroiditis, Behcet's disease, Crohn's disease, ulcerative colitis, bullous pemphigoid, sarcoidosis, psoriasis, ichthyosis, Graves ophthalmopathy, inflammatory bowel disease, Addison's disease, Vitiligo, asthma, or allergic asthma. Optionally, the autoimmune disease is arthritis, Crohn's disease, scleroderma, or rheumatoid arthritis. Optionally, the compound is a PTPRS de-clustering agent. Optionally, the PTPRS de-clustering agent is not chondroitin sulfate. Optionally, the de-clustering agent is not chondroitin sulfate, a chondroitin sulfate mimetic or an agent that has the same or similar mechanism of action as chondroitin sulfate. Optionally, the PTPRS de-clustering agent is an anti-PTPRS antibody or fragment thereof, an anti-heparan sulfate antibody, or a chondroitin sulfate mimetic. Optionally, as described throughout, the PTPRS de-clustering agent can be a non-enzymatic recombinant protein comprising an amino acid sequence of an extracellular domain of PTPRS or a subsequence, portion, homologue variant or derivative thereof.

The methods include administering an effective amount of the provided agents and compositions, wherein administering the effective amount of the composition treats or prevents the autoimmune disease in the subject. Administration of a composition disclosed herein can be a systemic or localized administration. For example, treating a subject having an inflammatory autoimmune disorder can include administering an oral or injectable form of the pharmaceutical composition on a daily basis or otherwise regular schedule. Optionally, the agents and composition are formulated for administration can be formulated for delivery to synovial fluid and/or for delivery to fibroblast-like synoviocytes. In some instances, the treatment is only on an as-needed basis, e.g., upon appearance of inflammatory autoimmune disease symptoms.

Also provided are methods of decreasing fibroblast activity in a subject. The methods include administering to the subject a therapeutically effective amount of a PTPRS de-clustering agent, wherein administration decreases fibroblast activity in the subject. Optionally, the de-clustering agent is not chondroitin sulfate, a chondroitin sulfate mimetic or an agent that has the same or similar mechanism of action as chondroitin sulfate. In some instances, the de-clustering agent is a chondroitin sulfate mimetic. Optionally, the PTPRS de-clustering agent is a non-enzymatic recombinant protein as provided herein. Optionally, the PTPRS de-clustering agent binds heparan sulfate. Optionally, the PTPRS de-clustering agent is an anti-PTPRS antibody or fragment thereof or an anti-heparan sulfate antibody or fragment thereof. Optionally, the fibroblast activity comprises fibroblast migration. Optionally, the fibroblast activity comprises collagen production, glycosaminoglycan production, reticular and elastic fiber production, cytokine production, chemokine production, glycoprotein production or combinations thereof. Optionally, the fibroblast activity comprises extracellular matrix production. Fibroblasts include, but are not limited to, synovial fibroblasts, dermal fibroblasts, and interstitial fibroblasts. Optionally, the fibroblasts are synovial fibroblasts.

Optionally, the subject has a fibroblast-mediated disease. Thus, provided are methods of treating a fibroblast mediated disease in a subject. The methods include administering to the subject a therapeutically effective amount of a PTPRS de-clustering agent, wherein administration treats the fibroblast-mediated disease in the subject. Optionally, the de-clustering agent is not chondroitin sulfate, a chondroitin sulfate mimetic or an agent that has the same or similar mechanism of action as chondroitin sulfate. In some instances, the de-clustering agent is a chondroitin mimetic. Optionally, the PTPRS de-clustering agent is a non-enzymatic recombinant protein as provided herein. Optionally, the PTPRS de-clustering agent binds heparan sulfate. Optionally, the PTPRS de-clustering agent is an anti-PTPRS antibody or fragment thereof or an anti-heparan sulfate antibody or fragment thereof. Fibroblast-mediated diseases include, but are not limited to, fibrosis and fibroblast-mediated autoimmune diseases. The fibrosis can be, for example, pulmonary fibrosis, idiopathic pulmonary fibrosis, liver fibrosis, endomyocardial fibrosis, atrial fibrosis, mediastinal fibrosis, myelofibrosis, retroperitoneal fibrosis, nephrogenic systemic fibrosis, skin fibrosis, or arthrofibrosis. The fibroblast-mediated autoimmune disease can be, for example, Crohn's disease, arthritis, rheumatoid arthritis, and scleroderma.

Provided herein are methods of modulating extracellular matrix in a subject, the method comprising administering to the subject an effective amount of the non-enzymatic recombinant protein provided herein, wherein administration modulates the extracellular matrix in the subject. Optionally, the method does not include administration of chondroitin sulfate, a chondroitin sulfate mimetic or an agent that has the same or similar mechanism of action as chondroitin sulfate. Modulation of the extracellular matrix includes, for example, modulation of one or more components of the extracellular matrix. Optionally, the extracellular matrix component is selected from the group consisting of a proteoglycan, polysaccharide or fiber. Optionally, the extracellular matrix component is a proteoglycan, e.g., heparan sulfate or chondroitin sulfate. Optionally, the extracellular matrix component is heparan sulfate. Optionally, the subject has an extracellular matrix disease. Extracellular matrix diseases are known and include, but are not limited to, atherosclerosis, cancer, an amyloid disease, an inflammatory condition, and a developmental disorder. Optionally, the amyloid disease is Alzheimer's disease or inflammation-related AA amyloidosis. Optionally, the inflammatory condition is osteoarthritis, systemic scleroderma, or lupus.

The herein provided methods that include the treatment of subjects with an inflammatory condition, autoimmune disease, fibroblast-mediated disease, or extracellular matrix disease can include administration of one or more additional agents that treat or prevent the inflammatory condition or autoimmune disease. For example, the provided methods can further include administration of and effective amount of one or more of anti-inflammatory agents. Suitable additional agents for use in the provided methods include, but are not limited to, analgesics, non-steroidal anti-inflammatory drugs, disease-modifying anti-rheumatic drugs, corticosteroids, and vitamin D analogues. Exemplary disease-modifying anti-rheumatic drugs for treating or preventing rheumatoid arthritis include, but are not limited to, azathioprine, cyclosporine A, D-penicillamine, gold salts, hydroxychloroquine, leflunomide, methotrexate (MTX), minocycline, sulfasalazine (SSZ), and cyclophosphamide.

Combinations of agents or compositions can be administered either concomitantly (e.g., as a mixture), separately but simultaneously (e.g., via separate intravenous lines) or sequentially (e.g., one agent is administered first followed by administration of the second agent). Thus, the term combination is used to refer to concomitant, simultaneous or sequential administration of two or more agents or compositions. The course of treatment is best determined on an individual basis depending on the particular characteristics of the subject and the type of treatment selected. The treatment, such as those disclosed herein, can be administered to the subject on a daily, twice daily, bi-weekly, monthly or any applicable basis that is therapeutically effective. The treatment can be administered alone or in combination with any other treatment disclosed herein or known in the art. The additional treatment can be administered simultaneously with the first treatment, at a different time, or on an entirely different therapeutic schedule (e.g., the first treatment can be daily, while the additional treatment is weekly).

According to the methods provided herein, the subject is administered an effective amount of one or more of the agents provided herein. The terms effective amount and effective dosage are used interchangeably. The term effective amount is defined as any amount necessary to produce a desired physiologic response (e.g., reduction of inflammation). Effective amounts and schedules for administering the agent may be determined empirically by one skilled in the art. The dosage ranges for administration are those large enough to produce the desired effect in which one or more symptoms of the disease or disorder are affected (e.g., reduced or delayed). The dosage should not be so large as to cause substantial adverse side effects, such as unwanted cross-reactions, anaphylactic reactions, and the like. Generally, the dosage will vary with the age, condition, sex, type of disease, the extent of the disease or disorder, route of administration, or whether other drugs are included in the regimen, and can be determined by one of skill in the art. The dosage can be adjusted by the individual physician in the event of any contraindications. Dosages can vary and can be administered in one or more dose administrations daily, for one or several days. Guidance can be found in the literature for appropriate dosages for given classes of pharmaceutical products. For example, for the given parameter, an effective amount will show an increase or decrease of at least 5%, 10%, 15%, 20%, 25%, 40%, 50%, 60%, 75%, 80%, 90%, or at least 100%. Efficacy can also be expressed as "-fold" increase or decrease. For example, a therapeutically effective amount can have at least a 1.2-fold, 1.5-fold, 2-fold, 5-fold, or more effect over a control. The exact dose and formulation will depend on the purpose of the treatment, and will be ascertainable by one skilled in the art using known techniques (see, e.g., Lieberman, Pharmaceutical Dosage Forms (vols. 1-3, 1992); Lloyd, The Art, Science and Technology of Pharmaceutical Compounding (1999); Remington: The Science and Practice of Pharmacy, 20th Edition, Gennaro, Editor (2003), and Pickar, Dosage Calculations (1999)).

Optionally, the provided methods of treatment or method of modulating PTPRS activity or function in a subject further includes obtaining a biological sample from the subject and determining whether the subject has an altered RNA level or an altered protein level of an PTPRS as compared to a control, an altered RNA level or altered protein level indicating the subject has or is at risk of developing an inflammatory condition, autoimmune disease, fibroblast-mediated disease or extracellular matrix disease. Optionally, the altered level is an elevated level as compared to a control. A control sample or value refers to a sample that serves as a reference, usually a known reference, for comparison to a test sample. For example, a test sample can be taken from a patient suspected of having autoimmune disease and compared to samples from a subject known to have an autoimmune disease or a known normal (non-disease) subject. A control can also represent an average value gathered from a population of similar individuals, e.g., autoimmune disease patients or healthy individuals with a similar medical background, same age, weight, etc. A control value can also be obtained from the same individual, e.g., from an earlier-obtained sample, prior to disease, or prior to treatment.

Thus, also provided are methods of determining whether a subject has or is at risk for developing an inflammatory condition, autoimmune disease, fibroblast-mediated disease or extracellular matrix disease comprising obtaining a biological sample from the subject and determining whether the subject has an elevated RNA level or an elevated protein level of an PTPRS, an elevated RNA level or elevated protein level indicating the subject has or is at risk of developing an autoimmune disease, inflammatory disease, fibroblast-mediated disease or extracellular matrix disease. Optionally, the provided methods further comprise selecting a subject with an autoimmune disease. Optionally, the autoimmune disease is an inflammatory autoimmune disease, e.g., arthritis or rheumatoid arthritis. As used herein, biological samples include, but are not limited to, cells, tissues and bodily fluids. Bodily fluids that used to evaluate the presence, absence or level of PTPRS RNA or protein include without limitation whole blood, plasma, urine, serum, tears, lymph, bile, cerebrospinal fluid, interstitial fluid, aqueous or vitreous humor, colostrum, sputum, amniotic fluid, saliva, a bronchioaviolar lavage sample, perspiration, transudate, exudate, and synovial fluid. Optionally, the biological sample is derived from a joint tissue or bodily fluid. Optionally, the provided methods further comprise isolating cells from the joint tissue or bodily fluid thereby forming an isolated cell sample. Such isolated cell samples can comprise synoviocytes, fibroblasts, hematopoetic cells, macrophages, leukocytes, T-cells or a combination thereof. Optionally, the synoviocytes are fibroblast-like synoviocytes or macrophage-like synoviocytes. Optionally, the isolated cell sample comprises fibroblast-like synoviocytes.

Methods for detecting and identifying nucleic acids and proteins and interactions between such molecules involve conventional molecular biology, microbiology, and recombinant DNA techniques within the skill of the art. Such techniques are explained fully in the literature (see, e.g., Sambrook, Fritsch & Maniatis, Molecular Cloning: A Laboratory Manual, Second Edition 1989, Cold Spring Harbor Laboratory Press, Cold Spring Harbor, N.Y.; Animal Cell Culture, R. I. Freshney, ed., 1986).

Methods for detecting RNA are largely cumulative with the nucleic acid detection assays and include, for example, Northern blots, RT-PCR, arrays including microarrays and sequencing including high-throughput sequencing methods. In some embodiments, a reverse transcriptase reaction is carried out and the targeted sequence is then amplified using standard PCR. Quantitative PCR (qPCR) or real time PCR (RT-PCR) is useful for determining relative expression levels, when compared to a control. Quantitative PCR techniques and platforms are known in the art, and commercially available (see, e.g., the qPCR Symposium website, available at qpersymposium.com). Nucleic acid arrays are also useful for detecting nucleic acid expression. Customizable arrays are available from, e.g., Affymatrix. Optionally, methods for detecting RNA include sequencing methods. RNA sequencing are known and can be performed with a variety of platforms including, but not limited to, platforms provided by Illumina, Inc., (La Jolla, CA) or Life Technologies (Carlsbad, CA). See, e.g., Wang, et al., Nat Rev Genet. 10(1):57-63 (2009); and Martin, Nat Rev Genet. 12(10):671-82 (2011).

Protein levels or concentration can be determined by methods standard in the art for quantitating proteins, such as Western blotting, ELISA, ELISPOT, immunoprecipitation, immunofluorescence (e.g., FACS), immunohistochemistry, immunocytochemistry, etc., as well as any other method now known or later developed for quantitating protein in or produced by a cell.

Also provided are methods of identifying a candidate PTPRS de-clustering agent, the method comprising contacting a test agent with clustered PTPRS peptides and detecting de-clustering of the PTPRS peptides, thereby identifying a candidate PTPRS de-clustering agent. Optionally, the method of identifying a candidate PTPRS de-clustering agent includes contacting a test agent with PTPRS and heparan sulfate and determining whether the test agent inhibits or reduces binding of the PTPRS to heparan sulfate, inhibition or reduction of binding indicating the test agent is a PTPRS de-clustering agent. Optionally, the test agent is a nucleic acid, peptide, antibody or small molecule. Optionally, the clustered PTPRS forms part of a cell membrane or cell. The methods of identifying PTPRS de-clustering agents may be performed *in vitro, in situ* or *in vivo.* A test agent can be identified as a PTPRS de-clustering agent using the methods described in the example below and others known to those of skill in the art. See, e.g., see Sambrook et al., Molecular Cloning: A Laboratory Manual, 3rd Ed., Cold Spring Harbor Press, Cold Spring Harbor, NY (2001). Thus, de-clustering of clustered PTPRS can be performed using any appropriate method known in the art. Various methods are available to assess whether an agent is effective at declustering PTPRS. For example, a cell-based readout assay for PTPRS function can be used. Declustering of PTPRS can be observed by detecting a decrease in tyrosine phosphorylation of PTPRS substrates or downstream signaling intermediates. Declustering can also be observed by detecting a decrease in migration or invasion of fibroblasts (e.g., FLS). Another assays include, but are not limited to, FRET-based assays, and gel-filtration. For example, in such assays, cells can be treated with HS to induce PTPRS clustering, and then treated with the test agents to test for declustering of PTPRS. Similarly, inhibition binding of PTPRS to heparan sulfate can be detected using any appropriate method known in the art. For example, an agent can be identified as an agent that inhibits or reduces binding of PTPRS to heparan sulfate by performing an assay in which binding of PTPRS to heparan sulfate can be detected (e.g., an immunoassay). The agent inhibits or reduces binding of PTPRS to heparan sulfate if binding of PTPRS to heparan sulfate can be detected in the absence of the agent but is no longer detected or binding is reduced in the presence of the agent. Optionally, binding can be detected by determining whether the agent to be tested competitively inhibits heparan sulfate from binding to PTPRS. Optionally, an agent can be identified as an agent that inhibits or reduces binding of PTPRS to heparan sulfate by performing an assay that measures the function or activity of PTPRS.

By way of example, a test agent can be identified as a PTPRS de-clustering agent by determining if the agent reduces the severity of one or more symptoms of the autoimmune or inflammatory disease or condition. Thus, by way of example, in the provided screening methods, the contacting step comprises administering the agent to a subject with an autoimmune or inflammatory disease and the determining step comprises determining whether the agent prevents or reduces one or more symptoms of the disease in a subject. Optionally, the disease is arthritis or rheumatoid arthritis. Such screening methods can be carried out using, for example, animal models of inflammatory and autoimmune disease, which can be obtained from commercial laboratories (e.g., Jackson Laboratory, 600 Main Street, Bar Harbor, Maine 04609 USA). Such screening methods can also be carried out using the mouse models and methods described in the example below.

Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutations of these compounds may not be explicitly disclosed, each is specifically contemplated and described herein. For example, if a method is disclosed and discussed and a number of modifications that can be made to a number of molecules including the method are discussed, each and every combination and permutation of the method, and the modifications that are possible are specifically contemplated unless specifically indicated to the contrary. Likewise, any subset or combination of these is also specifically contemplated and disclosed. This concept applies to all aspects of this disclosure including, but not limited to, steps in methods using the disclosed compositions. Thus, if there are a variety of additional steps that can be performed, it is understood that each of these additional steps can be performed with any specific method steps or combination of method steps of the disclosed methods, and that each such combination or subset of combinations is specifically contemplated and should be considered disclosed.

### Example

### Example 1. Functional interactions between PTPRS and proteoglycans in fibroblast-like synoviocytes.

Protein Tyrosine Phosphatase, Receptor Type, S or Sigma (PTPRS) has an extracellular domain that includes multiple fibronectin type III-like domains and immunoglobulin-like (Ig) domains. The two most external Ig domains (called Ig1 and Ig2) interact with nanomolar affinity with HS- (Heparan sulfate) and CS- (chondroitin-sulfate) containing PG (proteoglycan). The interaction of PTPRS with HS-PG (proteoglycan) (which are layered on the cell surface of most cell types) induces a clustered topology of PTPRS, which inhibits the phosphatase action and promotes signaling. On the contrary, CS-PG (enriched in the ECM surrounding neurons and other cells) effectively competes with HS-PG inducing a declustered conformation, which is active and leads to signaling inhibition. In certain experiments, PTPRS knockout mice were used. These mice showed a phenotype of decreased body weight and brain size, deficits in learning memory, nerve functions, increased spinal cord regeneration after hemisection and contusion injuries and increased axon formation through inhibitory barriers (CSPG).

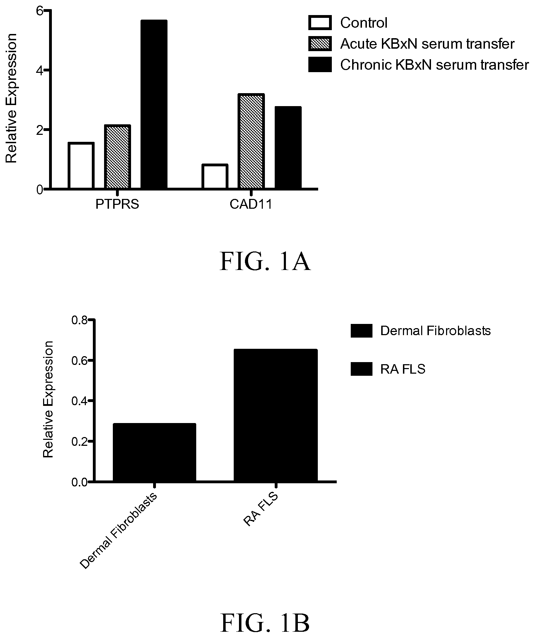

Mice that express the T cell receptor (TCR) transgene KRN and the MHC class II molecule A(g7) (K/BxN mice) develop severe inflammatory arthritis. The serum from these mice causes arthritis in other mouse strains. To determine whether PTPRS is involved in arthritis, expression of PTPRS in mice given K/BxN serum was evaluated. Figures 1A and 1B are graphs depicting expression of PTPRS in fibroblast-like synoviocytes (FLS). Figure 1A shows expression in mice. Primary CD90+CD11b-CD3-CD45- mFLS were isolated from joint tissue 7 days after acute K/BxN serum transfer (single administration of 150 µL serum), 21 days after chronic K/BxN serum transfer (single administration of 150 µL serum followed by 2 weekly boosts of 50 µL serum), or from control uninjected mice. Figure 1A shows expression levels of PTPRS and Cadherin-11 (CAD11) as measured by qPCR. To determine whether expression of PTPRS is elevated in humans, expression levels of PTPRS were assessed by qPCR from normal human dermal fibroblasts and cultured FLS isolated from a rheumatoid joint. The results are shown in Figure 1B. PTPRS is also elevated in FLS isolated from a rheumatoid join in a human.

Increased bone erosion severity in arthritis for PTPRS -/- mice as compared with PTPRS +/+ mice was observed. The results are shown in Figures 2A-2F. Mice were injected with 100 µL K/BxN serum intraperitoneally at 8-10 weeks of age (PTPRS-/- received proportionally less serum to reflect their decreased body weight). Ankle thickness was measured with digitals calipers and ankles and wrists were given a clinical arthritis score between 0 (no redness or swelling) and 4 (maximal swelling with digits involved) every two days for 14 days. See Figures 2A, 2B, and 2C. After 14 days, mice were euthanized and ankles fixed for histological scoring using H&E (inflammation and bone erosion) and safranin O (cartliage erosion) staining. See Figures 2D (inflammation), 2E (cartilage erosion), and 2F (bone erosion).

In order to further investigate the role of PTPRS, primary mouse FLS were isolated and evaluated. Knee, ankle and elbow joints were pooled from individual mice and minced in warm RPMI media under sterile conditions. Minced joints from each mouse were incubated in 40 mL warm RPMI media containing 0.5 mg/mL collagenase type IX for 2 hours at room temperature on a rocking platform. Cells and joint pieces were collected by centrifugation and resuspended in FLS medium (DMEM containing 10% FBS, 100 IU/mL penicillin/100 µg/mL streptomycin, 10 µg/mL gentamycin, 2 mM glutamine) and plated in T75 flasks for 3 days before changing media. Once cells reached -90% confluence, joint pieces were rinsed away and cells were subcultured. Cells were expanded and frozen down at passage 2 and were used for experiments between passages 4 and 8.

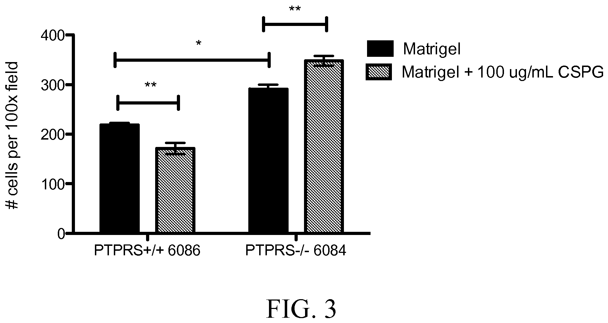

mFLS from PTPRS -/- mice were grown to -90% confluence and starved overnight. Transwell inserts (0.8 µm pore size) were coated with 100 µL matrigel diluted 1:25 in cold serum-free media containing no or 100 µg/mL chondroitin sulfate (CS) and incubated at 37°C for 2 hours before placing in 600 µL FLS media containing 5% FBS. 2.5 x 10⁴ cells were plated in each transwell chamber in 100 µL FLS media containing 0.5% BSA. Cells were allowed to invade for three days at 37°C, after which transwell inserts were rinsed with PBS and cells on the upper membrane were removed with a cotton swab. Cells that were the bottom membrane surface were fixed with methanol for 10 minutes and stained with crystal violet for 30 minutes. Cells were visualized using a Nikon 80i light microscope and five 100x fields per membrane were used for counting. The results are shown in Figure 3. The graph is representative of three independent experiments; three wells per treatment; cell lines from female littermates. Figure 3 shows that PTPRS -/- FLS exhibit reduced CS-mediated inhibition of invasion.

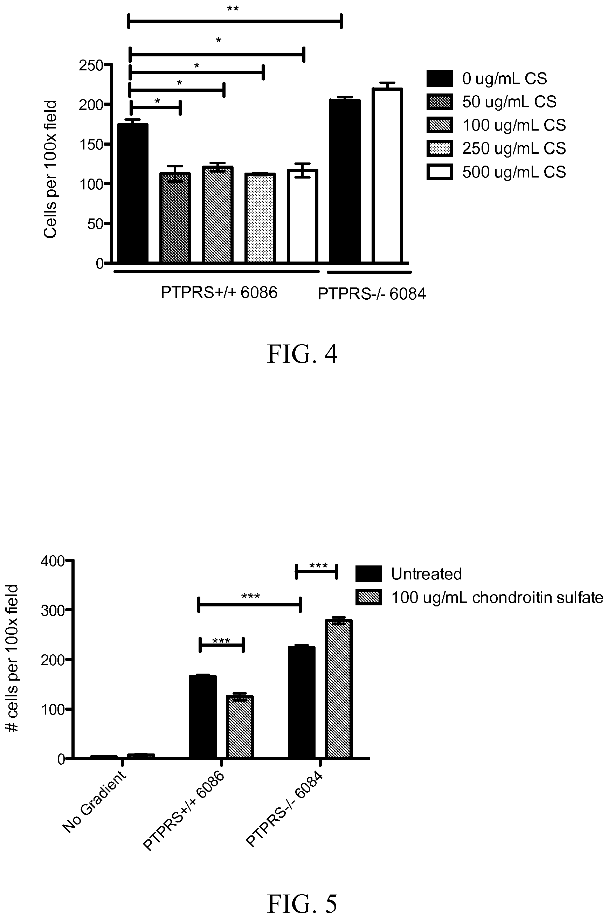

mFLS from PTPRS +/+ and PTPRS -/- mice were grown to -90% confluence and starved overnight. Transwell inserts (0.8 µm pore size) were pre-wet with serum-free FLS media at 37°C for 30 minutes before placing in 600 µL FLS media containing 5% FBS. 2.5 x 10⁴ cells were plated in each transwell chamber in 100 µL FLS media containing 0.5% BSA and increasing concentrations of chondroitin sulfate (CS). Cells were allowed to migrate for 4 hours at 37°C, after which transwell inserts were rinsed with PBS and cells on the upper membrane were removed with a cotton swab. Cells migrating to the bottom membrane surface were fixed with methanol for 10 minutes and stained with crystal violet for 30 minutes. Cells were visualized using a Nikon 80i light microscope and five 100x fields per membrane were used for counting. The results are shown in Figure 4. The asterisk signifies statistical significant difference between the two samples. Figure 4 shows that PTPRS-/- mFLS are insensitive to CS at levels approximately 5 times higher than those in the joint.

mFLS from PTPRS +/+ and PTPRS -/- mice were grown to -90% confluence and starved overnight. Transwell inserts (0.8 µm pore size) were pre-wet with serum-free FLS media at 37°C for 30 minutes before placing in 600 µL FLS media containing 5% FBS. 2.5 x 10⁴ cells were plated in each transwell chamber in 100 µL FLS media containing 0.5% BSA with or without 100 µg/mL chondroitin sulfate (CS). Cells were allowed to migrate for 4 hours at 37°C, after which transwell inserts were rinsed with PBS and cells on the upper membrane were removed with a cotton swab. Cells migrating to the bottom membrane surface were fixed with methanol for 10 minutes and stained with crystal violet for 30 minutes. Cells were visualized using a Nikon 80i light microscope and five 100x fields per membrane were used for counting. The results are shown in Figure 5, which are representative of three independent experiments; three wells per treatment; cell lines from female littermates; confirmed in second independent pair of male cell lines. Figure 5 shows that PTPRS-/- mFLS exhibit reduced CS-mediated inhibition of migration.

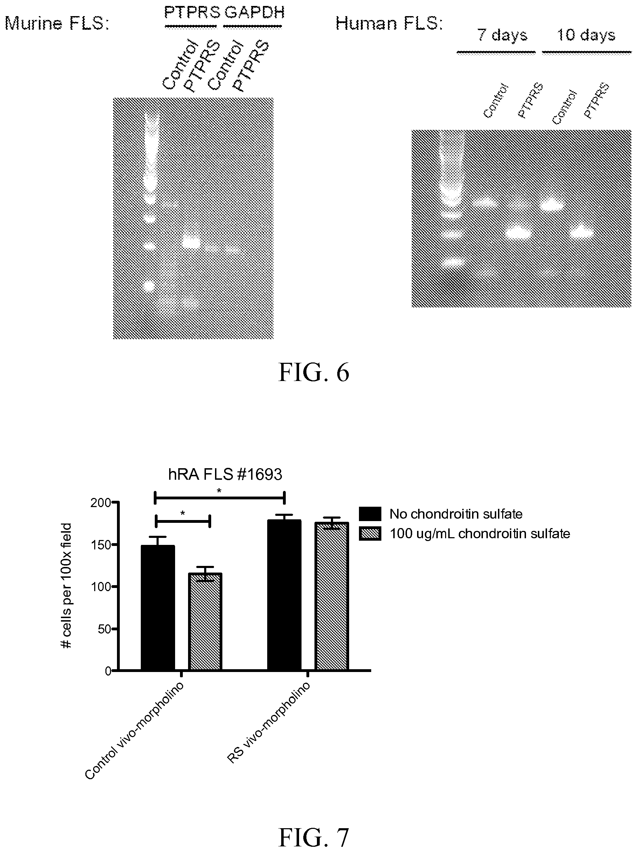

Cell permeable morpholino antisense oligos were obtained from GeneTools (Philomath, OR) and were designed to bind intron-exon junctions and cause the splicing of a selected exon resulting in a frameshift. This ultimately causes a truncated, non-functional mRNA product after 7 days. The results are shown in Figure 6. The morpholinos mediated knockdown of PTPRS in mouse and human primary FLS.

To determine the effect of PTPRS knockdown in human rheumatoid arthritis (RA), hRA FLS were seeded in 6-well plates and allowed to adhere overnight. Media was replaced containing 2.5 µM morpholino oligo (either control or directed against PTPRS) and knockdown was achieved after 7 days (changing media and oligo at day 3). Cells were then starved overnight prior to migration assay. Transwell inserts (0.8 µm pore size) were pre-wet with serum-free FLS media at 37°C for 30 minutes before placing in 600 µL FLS media containing 5% FBS. 5 x 10⁴ cells were plated in each transwell chamber in 100 µL FLS media containing 0.5% BSA with or without 100 µg/mL chondroitin sulfate (CS). Cells were allowed to migrate for 24 hours at 37°C, after which transwell inserts were rinsed with PBS and cells on the upper membrane were removed with a cotton swab. Cells migrating to the bottom membrane surface were fixed with methanol for 10 minutes and stained with crystal violet for 30 minutes. Cells were visualized using a Nikon 80i light microscope and five 100x fields per membrane were used for counting. The results are shown in Figure 7, which are representative of five independent experiments in three patient cell lines; three wells per treatment. Figure 7 shows PTPRS knockdown increases migration of human RA FLS and abrogates CS-mediated inhibition of migration.

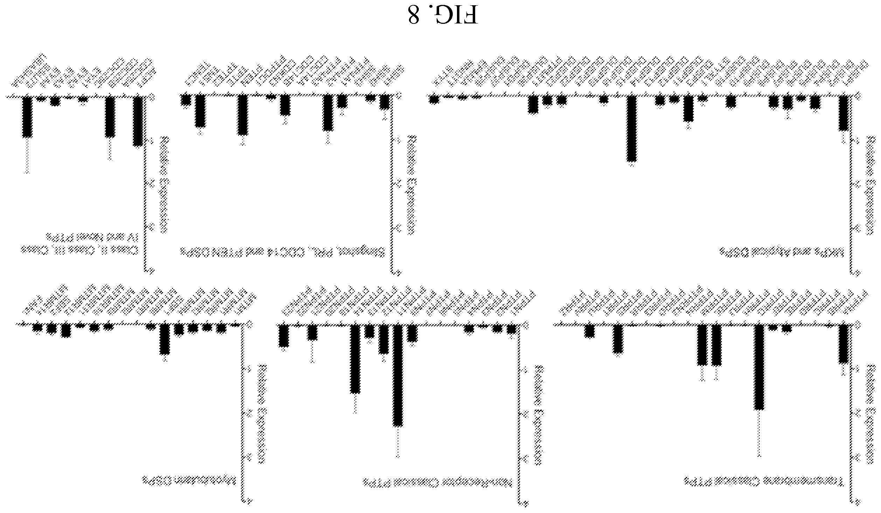

To investigate further the role of PTPs in autoimmune disease, the mRNA PTPome of RA FLS was analyzed. The results are shown in Figure 8. At least 5 PTPs were highly expressed in RA FLS, one of which was PTPRS.

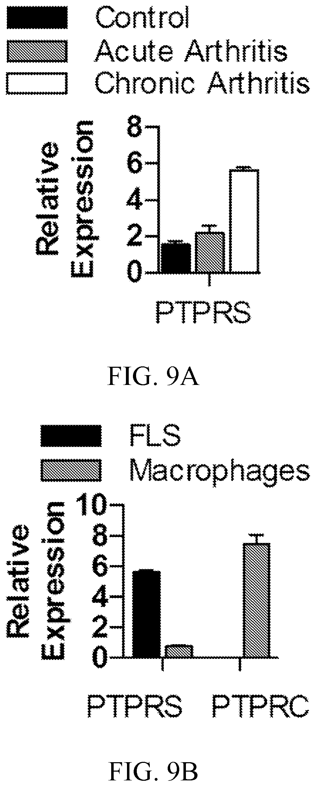

To further study the expression of PTPRS, expression in FLS and macrophages from pooled mice was measured by quantitative polymerase chain reaction. The results are shown in Figures 9A and 9B. Values are the mean of PTP expression relative to the housekeeping gene GAPDH. Figure 9A shows FLS were sorted from control mice or mice with K/BxN serum induced acute or chronic arthritis and Figure 9B shows mouse sorted macrophages and FLS with PTPRC included as a positive control for expression in macrophages.

To further study the effects of PTPRS on FLS migration, PTPRS WT or KO FLS were allowed to invade through Matrigel-coated transwell chambers. Cells per field were counted after invasion for 24 hours in response to 5% fetal bovine serum (FBS) in the presence of chondroitin sulfate (CS). The results are shown in Figure 10A. In Figure 10B, PTPRS WT or KO FLS were allowed to migrate through uncoated transwell chambers. Cells per field were counted after migration for 4 hours in response to 5% FBS in the presence of CS with or without chondroitinase ABC treatment. Bars indicate mean +/- standard error of the fold-change of migration relative to that of untreated cells. Data include three independent experiments using three sets of littermate cell lines. (***P < 0.001; significance determined using Mann-Whitney test on raw data). Figures 10A and 10B show that PTPRS KO mice FLS did not migrate in response to CS.

As shown in Figures 11A and 11B, the inhibition of RA FLS migration by chondroitin sulfate (CS) is dependent on PTPRS expression. Figure 11A is a graph of RT-PCR data showing knock-down of PTPRS in RA FLS after incubation with anti-sense oligo (ASO) for 7 days. In Figure 11B, PTPRS WT or KO FLS were allowed to migrate through uncoated transwell chambers. Cells per field were counted after migration for 4 h in response to 5% fetal bovine serum (FBS) in the presence of CS with or without chondroitinase ABC treatment. Bars indicate mean +/- standard error of the fold-change of migration relative to that of untreated cells. Data include three independent experiments using three sets of littermate cell lines. (***P < 0.001; significance determined using Mann-Whitney test on raw data).

As shown in Figures 12A and 12B, displacement of PTPRS from heparin sulfate (HS) is sufficient to inhibit FLS migration and invasion. PTPRS WT or KO FLS were allowed to invade through uncoated transwell chambers. Cells per field were counted after migration for 4 h in response to 5% fetal bovine serum (FBS) in the presence of 20 nM Ig1+2 alone (Figure 12A) or in addition to 100 µg/mL CS (Figure 12B). Bars indicate mean +/- standard error of the fold-change of migration relative to that of untreated cells. Data include three independent experiments using three sets of littermate cell lines. (**P < 0.01, ***P < 0.001, ****P < 0.0001; significance determined using Mann-Whitney test on raw data).

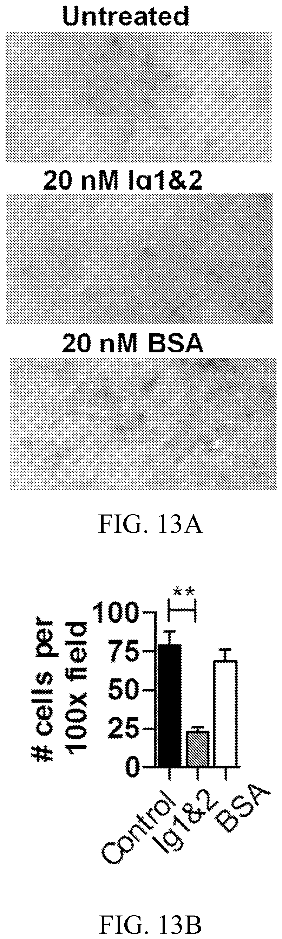

To study the effects of Ig1 and 2 on bovine cartilage, light micrographs of bovine cartilage explants were taken in the presence and absence of Ig1+2 treatment. Figure 13A shows light micrographs stained with Mayer's hematoxylin with and without Ig1+2 treatments. Figure 13B is a graph showing cell counts with and without Ig1+2 treatments.

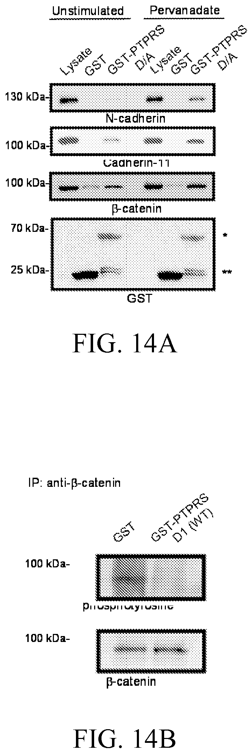

Figures 14A and 14B are graphs showing beta-catenin and cadherin binding and desphosphorylation. Figure 14A is a Western blot of total cell lysates or lysates incubated with GST or GST-PTPRS D/A substrate trapping mutant from unstimulated or pervanadate stimulated FLS. * represents GST-PTPRS D1 D/A and ** represents GST tag. Figure 14B Western blot showing decreased tyrosine phosphorylation of immunopurified beta-catenin in the presence of GST-PTPRS D1 compared to GST alone.

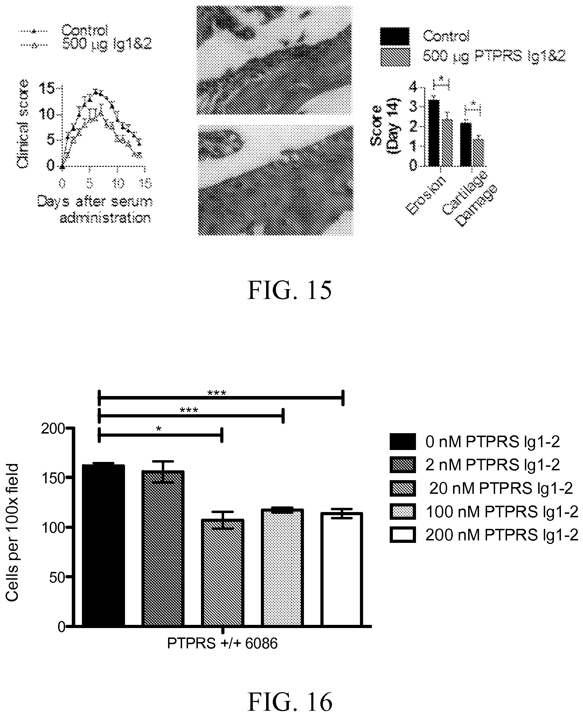

Figure 15 is a graph showing the effect of Ig1 and Ig2 in ameliorating arthritis. Clinical Score of BALB/c mice induced with acute arthritis by administration of 100 µL K/BxN serum on Day 0 and treated with vehicle (control) or 500 µg Ig1+2 daily from days 0 to 6. N=3 per treatment. Histopathological analysis of the joint with and without Ig1+2 treatment plus erosion and cartilage damage score.

As shown in Figure 16, treatment of mFLS with recombinant PTPRS Ig1+2 reduces migration. mFLS were grown to -90% confluence and starved overnight. Transwell inserts (0.8 µm pore size) were pre-wet with serum-free FLS media at 37°C for 30 minutes before placing in 600 µL FLS media containing 5% FBS. 2.5 x 10⁴ cells were plated in each transwell chamber in 100 µL FLS media containing 0.5% BSA with increasing concentrations of recombinant mouse PTPRS Ig1+2 domains. Cells were allowed to migrate for 4 hours at 37°C, after which transwell inserts were rinsed with PBS and cells on the upper membrane were removed with a cotton swab. Cells migrating to the bottom membrane surface were fixed with methanol for 10 minutes and stained with crystal violet for 30 minutes. Cells were visualized using a Nikon 80i light microscope and five 100x fields per membrane were used for counting. Figure 16 shows that recombinant PTPRS Ig1+2 reduces migration of FLS.

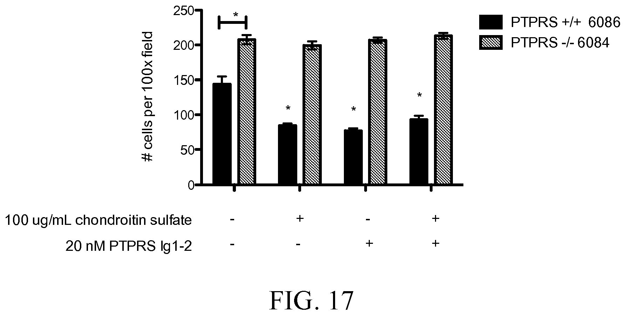

As shown in Figure 17, PTPRS-/- mFLS are insensitive to recombinant PTPRS Ig1+2 mediated reduced migration. mFLS were grown to -90% confluence and starved overnight. Transwell inserts (0.8 µm pore size) were pre-wet with serum-free FLS media at 37°C for 30 minutes before placing in 600 µL FLS media containing 5% FBS. 2.5 x 10⁴ cells were plated in each transwell chamber in 100 µL FLS media containing 0.5% BSA and in the presence or absence of 20 nM recombinant mouse PTPRS Ig1+2 domains and/or 100 µg/mL chondroitin sulfate (CS). Cells were allowed to migrate for 4 hours at 37°C, after which transwell inserts were rinsed with PBS and cells on the upper membrane were removed with a cotton swab. Cells migrating to the bottom membrane surface were fixed with methanol for 10 minutes and stained with crystal violet for 30 minutes. Cells were visualized using a Nikon 80i light microscope and five 100x fields per membrane were used for counting. The asterisk in Figure 17 indicates three wells per treatment and cell lines from female littermates. Figure 17 shows that PTPRS-/- mFLS do not respond to PTPRS Ig1+2 treatment.

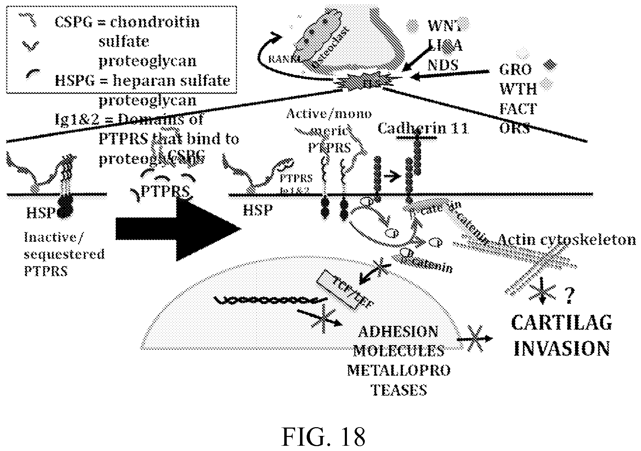

The example and data provided herein demonstrate that PTPRS is highly expressed in FLS and binds chondroitin sulfate (CS) with high affinity. Further, PTPRS mediates the inhibitory action of CS on FLS migration and invasion and recombinant CS-binding domain of PTPRS replicates the action of CS at nanomolar concentrations. This data suggest that proposed model of competition between CS and HS for PTPRS binding is correct. See Figure 18. This data further suggest that PTPRS is normally mostly in inactive conformation due to heavy binding to HS and that displacement of HS using recombinant Ig1+2 or CS-mimicking antibodies enables activation of the phosphatase. PTPRS is expressed at low levels in hemopoietic cells and KO mouse has no hemopoietic phenotype suggesting anti-PTPRS therapy would be minimally immunosuppressive.

## Claims

1. A non-enzymatic recombinant protein comprising an amino acid sequence of an extracellular domain of PTPRS, wherein the protein comprises both PTPRS immunoglobulin-like domain 1 (Ig1) and immunoglobulin-like domain 2 (Ig2), for use in therapy.

2. The non-enzymatic recombinant protein for use of claim 1, wherein the extracellular domain of PTPRS comprises PTPRS immunoglobulin-like domain 1 (Ig1), immunoglobulin-like domain 2 (Ig2) and immunoglobulin-like domain 3 (Ig3).

3. The non-enzymatic recombinant protein for use of claim 1 or 2, wherein the protein comprises Ig1 amino acid residues 30 to 127 of SEQ ID NO:4 or amino acid residues 30-127 of SEQ ID NO:8.

4. The non-enzymatic recombinant protein for use of claim 3, wherein the protein comprises an amino acid sequence set forth as:
EEPRFIKEPKDQIGVSGGVASFVCQATGDPKPRVTWNKKGKKVNSQRFETIEFDESA GAVLRIQPLRTPRDENVYECVAQNSVGEITVHAKLTVLRE (SEQ ID NO:1) or as set forth in SEQ ID NO:5.

5. The non-enzymatic recombinant protein for use of claim 1 or 2, wherein the protein comprises Ig2 amino acid residues 128 to 231 of SEQ ID NO:4 or amino acid residues 128-244 of SEQ ID NO:8.

6. The non-enzymatic recombinant protein for use of claim 5 wherein the protein comprises an amino acid sequence set forth as:
DQLPSGFPNIDMGPQLKVVERTRTATMLCAASGNPDPEITWFKDFLPVDPSASNGRI KQLRSETFESTPIRGALQIESSEETDQGKYECVATNSAGVRYSSPANLYVRVRRVA (SEQ ID NO:2) or as set forth in SEQ ID NO:6.

7. The non-enzymatic recombinant protein for use of claim 1 or 2, wherein the protein comprises Ig3 amino acid residues 232-321 of SEQ ID NO:4 or amino acid residues 245-334 of SEQ ID NO:8.

8. The non-enzymatic recombinant protein for use of claim 7, wherein the protein comprises an amino acid sequence set forth as:
PRFSILPMSHEIMPGGNVNITCVAVGSPMPYVKWMQGAEDLTPEDDMPVGRNVLEL TDVKDSANYTCVAMSSLGVIEAVAQITVKSLPKA (SEQ ID NO:3) or as set forth in SEQ ID NO:7.

9. The non-enzymatic recombinant protein for use of any one of claims 1 to 8, wherein the protein binds heparan sulfate.

10. The non-enzymatic recombinant protein for use as defined in any one of claims 1 to 9 for use in the treatment of a subject who has arthritis.

11. The non-enzymatic recombinant protein for use of claim 10 wherein the arthritis is rheumatoid arthritis.

12. A PTPRS de-clustering agent for use in a method of treating arthritis wherein the PTPRS de-clustering agent is selected from: the non-enzymatic recombinant protein as defined in any one of claims 1 to 10; an anti-PTPRS antibody or fragment thereof; or an anti-heparan sulfate antibody, the method comprising administering to the subject a therapeutically effective amount of the PTPRS de-clustering agent, wherein administration treats arthritis in the subject.

13. The PTPRS de-clustering agent for use of claim 12, wherein the arthritis is rheumatoid arthritis.

## Patentansprüche

1. Nicht-enzymatisches rekombinantes Protein, das eine Aminosäuresequenz einer extrazellulären Domäne von PTPRS aufweist, wobei das Protein sowohl die PTPRS-Immunglobulin-ähnliche Domäne 1 (Ig1) als auch die Immunglobulin-ähnliche Domäne 2 (Ig2) umfasst, zur Verwendung in der Therapie.

2. Nicht-enzymatisches rekombinantes Protein nach Anspruch 1, wobei die extrazelluläre Domäne von PTPRS die PTPRS-Immunglobulin-ähnliche Domäne 1 (Ig1), die Immunglobulin-ähnliche Domäne 2 (Ig2) und die Immunglobulin-ähnliche Domäne 3 (Ig3) umfasst.

3. Nicht-enzymatisches rekombinantes Protein zur Verwendung nach Anspruch 1 oder 2, wobei das Protein die Ig1-Aminosäurereste 30 bis 127 von SEQ ID NO:4 oder die Aminosäurereste 30-127 von SEQ ID NO:8 umfasst.

4. Nicht-enzymatisches rekombinantes Protein zur Verwendung nach Anspruch 3, wobei das Protein eine Aminosäuresequenz umfasst, die wie folgt angegeben ist:
EEPRFIKEPKDQIGVSGGGVASFVCQATGDPKPRVTWNKKKG KKVNSQRFETIEFDESAGAVLRIQPLRTPRDENVYECVAQNSVGEIT VHAKLTVLRE (SEQ ID NO:1) oder wie in SEQ ID NO:5 angegeben ist.

5. Nicht-enzymatisches rekombinantes Protein zur Verwendung nach Anspruch 1 oder 2, wobei das Protein die Ig2-Aminosäurereste 128 bis 231 von SEQ ID NO:4 oder die Aminosäurereste 128-244 von SEQ ID NO:8 umfasst.

6. Nicht-enzymatisches rekombinantes Protein zur Verwendung nach Anspruch 5, wobei das Protein eine Aminosäuresequenz umfasst, die wie folgt angegeben ist:
DQLPSGFPNIDMGPQLKVVERTRTATMLCAASGNPDPElTWFK DFLPVDPSASNGRIKQLRSETFESTPIRGALQIESSEETDQGKYECV ATNSAGVRYSSPANLYVRVRRVA (SEQ ID NO:2), oder die wie in SEQ ID NO:6 angegeben ist.

7. Nicht-enzymatisches rekombinantes Protein zur Verwendung nach Anspruch 1 oder 2, wobei das Protein die Ig3-Aminosäurereste 232-321 von SEQ ID NO:4 oder die Aminosäurereste 245-334 von SEQ ID NO:8 umfasst.

8. Nicht-enzymatisches rekombinantes Protein zur Verwendung nach Anspruch 7, wobei das Protein eine Aminosäuresequenz umfasst, die wie folgt angegeben ist:
PRFSILPMSHEIMPGGGNVNITCVAVGSPMPYVKWMQGAEDL TPEDDMPVGRNVLELTDVKDSANYTCVAMSSLGVIEAVAVAQITVK SLPKA (SEQ ID NO:3), oder die wie in SEQ ID NO:7 angegeben ist.

9. Nicht-enzymatisches rekombinantes Protein zur Verwendung nach einem der Ansprüche 1 bis 8, wobei das Protein Heparansulfat bindet.

10. Nicht-enzymatisches rekombinantes Protein zur Verwendung nach einem der Ansprüche 1 bis 9 zur Verwendung bei der Behandlung eines Patienten mit Arthritis.

11. Nicht-enzymatisches rekombinantes Protein zur Verwendung nach Anspruch 10, wobei die Arthritis rheumatoide Arthritis ist.

12. PTPRS-Declustering-Mittel zur Verwendung in einem Verfahren zur Behandlung von Arthritis, wobei das PTPRS-Declustering-Mittel ausgewählt ist aus: dem nicht-enzymatischen rekombinanten Protein nach einem der Ansprüche 1 bis 10; einem Anti-PTPRS-Antikörper oder einem Fragment davon; oder einem Anti-Heparansulfat-Antikörper, wobei das Verfahren die Verabreichung einer therapeutisch wirksamen Menge des PTPRS-De-Clustering-Mittels an das Subjekt umfasst, wobei die Verabreichung Arthritis in dem Subjekt behandelt.

13. PTPRS-Declustering-Mittel zur Verwendung nach Anspruch 12, wobei die Arthritis rheumatoide Arthritis ist.

## Revendications

1. Protéine recombinante non enzymatique comprenant une séquence d'acides aminés d'un domaine extracellulaire de PTPRS, où la protéine comprend à la fois un domaine de type immunoglobuline 1 (Ig1) et un domaine de type immunoglobuline 2 (Ig2) de PTPRS, pour utilisation en thérapie.

2. Protéine recombinante non enzymatique pour utilisation selon la revendication 1, dans laquelle le domaine extracellulaire de PTPRS comprend le domaine de type immunoglobuline 1 (Ig1), le domaine de type immunoglobuline 2 (Ig2) et le domaine de type immunoglobuline 3 (Ig3) de PTPRS.

3. Protéine recombinante non enzymatique pour utilisation selon la revendication 1 ou 2, où la protéine comprend les résidus d'acides aminés 30 à 127 d'Ig1 de la SEQ ID NO : 4 ou les résidus d'acides aminés 30 à 127 de la SEQ ID NO : 8.

4. Protéine recombinante non enzymatique pour utilisation selon la revendication 3, où la protéine comprend une séquence d'acides aminés représentée en tant que :
EEPRFIKEPKDQIGVSGGVASFVCQATGDPKPRVTWNKKGKKVNSQRFETIEFD ESAGAVLRIQPLRTPRDENVYECVAQNSVGEITVHAKLTVLRE (SEQ ID NO : 1) ou telle que représentée dans la SEQ ID NO : 5.

5. Protéine recombinante non enzymatique pour utilisation selon la revendication 1 ou 2, où la protéine comprend les résidus d'acides aminés 128 à 231 d'Ig2 de la SEQ ID NO : 4 ou les résidus d'acides aminés 128 à 244 de la SEQ ID NO : 8.

6. Protéine recombinante non enzymatique pour utilisation selon la revendication 5, où la protéine comprend une séquence d'acides aminés représentée en tant que :
DQLPSGFPNIDMGPQLKVVERTRTATMLCAASGNPDPEITWFKDFLPVDPSASN GRIKQLRSETFESTPIRGALQIESSEETDQGKYECVATNSAGVRYSSPANLYVRVRRVA (SEQ ID NO : 2) ou telle que représentée dans la SEQ ID NO : 6.

7. Protéine recombinante non enzymatique pour utilisation selon la revendication 1 ou 2, où la protéine comprend les résidus d'acides aminés 232 à 321 d'Ig3 de la SEQ ID NO : 4 ou les résidus d'acides aminés 245 à 334 de la SEQ ID NO : 8.

8. Protéine recombinante non enzymatique pour utilisation selon la revendication 7, où la protéine comprend une séquence d'acides aminés représentée en tant que :
PRFSILPMSHEIMPGGNVNITCVAVGSPMPYVKWMQGAEDLTPEDDMPVGRNVL ELTDVKDSANYTCVAMSSLGVIEAVAQITVKSLPKA (SEQ ID NQ : 3) ou telle que représentée dans la SEQ ID NO : 7.

9. Protéine recombinante non enzymatique pour utilisation selon l'une quelconque des revendications 1 à 8, où la protéine se lie au sulfate d'héparane.

10. Protéine recombinante non enzymatique pour utilisation telle que définie dans l'une quelconque des revendications 1 à 9 pour utilisation dans le traitement d'un sujet qui souffre d'arthrite.

11. Protéine recombinante non enzymatique pour utilisation selon la revendication 10, où l'arthrite est la polyarthrite rhumatoïde.

12. Agent de dégroupement de PTPRS pour utilisation dans un procédé de traitement de l'arthrite où l'agent de dégroupement de PTPRS est choisi parmi : la protéine recombinante non enzymatique telle que définie dans l'une quelconque des revendications 1 à 10 ; un anticorps anti-PTPRS ou un fragment de celui-ci ; et un anticorps anti-sulfate d'héparane, le procédé comprenant l'étape consistant à administrer au sujet une quantité thérapeutiquement efficace de l'agent de dégroupement de PTPRS, où l'administration traite l'arthrite chez le sujet.

13. Agent de dégroupement de PTPRS pour utilisation selon la revendication 12, où l'arthrite est la polyarthrite rhumatoïde.
